# EUROPEAN PATENT APPLICATION

(11) **EP 2 966 177 A1**
(43) Date of publication of application: **13.01.2016**
(21) Application number: 14382266.6
(22) Date of filing: 09.07.2014
(51) Int. Cl.: C12Q 1/68

(54) **Methods for detecting target DNA sequences**

(71) Applicant: Vetgenomics, S.L., 08193 Bellaterra - Barcelona (ES)
(72) Inventor: Merkoçi Hyka, Arben, 08290 Cerdanyola del Vallés - Barcelona (ES); De La Escosura Muñiz, Alfredo, 08224 Terrassa - Barcelona (ES); Serrano Peralta, Lorena, 08027 Barcelona (ES); Altet Sanahujes, Laura, 08781 Els Hostalets de Pierola - Barcelona (ES); Francino Martí, Olga, 08195 Sant Cugat del Valles - Barcelona (ES)
(74) Representative: ABG Patentes, S.L.

(57) **Abstract**

The invention relates to methods for detecting target DNA sequences, particularly for detecting *Leishmania* infection, comprising a recombinase-polymerase isothermal amplification and detection by lateral flow or electrochemistry. The invention also relates to kits and oligonucleotides for performing said methods.

## Description

### FIELD OF THE INVENTION

The invention relates to methods for detecting target DNA sequences, specifically to the field of diagnostics and, more particular, to methods for detecting *Leishmania* infection comprising a recombinase-polymerase isothermal amplification and detection by lateral flow or electrochemistry.

### BACKGROUND OF THE INVENTION

The detection of specific DNA target sequences present in air, food, water, environment or clinical samples is of great significance in the medical, veterinary, microbiology, food and water safety-testing, and environment monitoring fields. The detection for the presence of a specific genetic sequence in a sample can rapidly and correctly identify genetic defects, oncogenic events and bacterial, viral or parasitic agents of concern.

In the field of diagnostics of infectious diseases accurate identification of the causative organism is time-critical. Some rapid, sensitive and specific methods have been developed to replace the time-consuming and limited culture methods.

Diseases transmitted by blood-feeding vectors (parasites) are a growing threat to world health, particularly those affecting pets and transmitted by fleas, ticks, sandflies or mosquitoes (Vector Borne Diseases, VBD). VBD are known by worldwide veterinarians and usually have zoonotic consequences being important for human health.

Pets usually have a main role in the maintenance of zoonotic infections. Major VBD are provoked by *Leishmania, Babesia, Anaplasma, Ehrlichia, Dirofilaria, Borrelia* (Lyme disease), *Rickettsia* and *Hepatozoon.* Several of these are global infections and may cause severe diseases in humans. Pets may play an important role as parasite reservoirs and as sentinels of the infection due to their contact with humans.

One of the most important vector-borne diseases is visceral leishmaniasis which is endemic in 88 countries on 4 continents. The overall prevalence is 12 million people although the population at risk is 350 million. Leishmaniasis is regarded as the most important zoonotic disease within Europe. Zoonotic visceral leishmaniasis, caused by the protozoan *Leishmania infantum* and transmitted by sandfly vectors, is a fatal disease of domestic dogs, wild canids and humans which occurs mainly in the Mediterranean, Middle East and South America. The domestic dog is considered the major host for zoonotic leishmaniasis mainly in Europe. Moreover, leishmaniasis is emerging within non endemic areas and is thus increasingly becoming an important concern both from human public health and animal welfare perspectives due to movement of infected dogs, ongoing climatic change and the consequent extension of the range of the sandfly vector towards more northern latitudes.

Guidelines for canine Leishmaniasis diagnostic include clinical signs, clinicopathological abnormalities and serological status being molecular test of limited use for the need of skilled workers, the high cost and the fact that samples must be send to a reference lab. Moreover, the presence of lower antibody levels is not necessarily indicative of disease and further work-up is necessary to confirm it by other diagnostic methods, being PCR the gold standard.

Several molecular tests capable of direct parasite detection in an inexpensive, quantitative, reliable and friendly to use format to be performed at the clinician (point-of-care, POC) have been developed.

A dipstick format was developed for detection of PCR products by hybridization with a gold-conjugate probe, and a PCR oligochromatographic leishmania-specific test is being commercialized using this technology (Leishmania OligoC-test, Coris Bioconcept) (Deborggraeve S. et al. 2008. JID, 198:1565-1572). In 2011, the US Food and Drug Administration (FDA) approved the Smart Leish PCR assay (Cepheid USA), a qualitative real-time PCR test for diagnosing individuals with cutaneous leishmaniasis. Both systems did not overcome the limitations of PCR for which sophisticated and expensive equipment is needed to perform the precise and repeated heating cycles required.

Mohan (Mohan S. et al. 2011. Analyst, 136: 2845-2851) discloses an electrochemical detection assay for *L.donovani* wherein the genomic DNA of *Leishmania* is extracted and hybridized with a single strand oligonucleotide specific for *L.donovani* that is immobilized onto an ITO/NiO electrode. However, this method has low sensitivity and does not allow detecting double-stranded PCR amplicons.

Therefore, there is still a need for further POC diagnostic methods for detecting infections by pathogens, particularly for diagnosing leishmaniasis.

### BRIEF DESCRIPTION OF THE INVENTION

In a first aspect, the invention relates to an *in vitro* method for detecting a target DNA sequence in a sample comprising the following steps:
a) submitting the sample DNA to a recombinase-polymerase isothermal nucleic acid amplification in the presence of a pair of primers capable of specifically amplifying the target DNA sequence wherein the first primer is labelled at the 5' end with a gold nanoparticle and the second primer is labelled at the 5' end with a first member of a binding pair, and
b) measuring the electrochemical signal produced by the gold nanoparticles forming part of the amplification products obtained in step a) using a screen-printed carbon electrode after capture of the amplification products on the electrode using magnetic beads conjugated to a second member of a binding pair,
wherein the detection of an electrochemical signal superior to the background signal indicates the presence of the target DNA sequence in the sample.

In a second aspect, the invention relates to an *in vitro* method for detecting a target DNA sequence in a sample comprising the following steps:
a) submitting the sample DNA to a recombinase-polymerase isothermal nucleic acid amplification in the presence of a pair of primers capable of specifically amplifying the target DNA sequence wherein the first primer is labelled at the 5' end with a first member of a first binding pair and the second primer is labelled at the 5' end with a first member of a second binding pair, and
   wherein said first members of the first and second binding pairs do not substantially cross-react with any second member of any different binding pair, and
b) measuring the electrochemical signal produced by the gold nanoparticles forming part of the amplification products obtained in step a) using a screen-printed carbon electrode after the first member of a first binding pair is bound to a second member of a first binding pair conjugated to a gold nanoparticle and after capture of the amplification products on the electrode using magnetic beads conjugated to a second member of a second binding pair
wherein the detection of an electrochemical signal superior to the background signal indicates the presence of the target DNA sequence in the sample.

In a third aspect, the invention relates to an *in vitro* method for detecting a target DNA sequence in a sample comprising the following steps:
a) submitting the sample DNA to a recombinase-polymerase isothermal nucleic acid amplification in the presence of
   (i) a first pair of primers capable of specifically amplifying the target DNA sequence wherein the first primer of the first pair of primers is labelled at the 5' end with a first member of a first binding pair and the second primer of the first pair or primers is labelled at the 5' end with a first member of a second binding pair and
   (ii) a second pair of primers capable of amplifying an endogenous amplification control DNA sequence present in the sample wherein the first primer of the second pair of primers is labelled at the 5' end with said first member of said first binding pair and the second primer of the second pair of primers is labelled at the 5' end with a first member of a third binding pair
      wherein said first members of the first, second and third binding pairs do not substantially cross-react with any second member of any different binding pair and
b) performing a lateral flow assay for the detection of the amplified DNA obtained in step a), wherein
   the amplified DNA obtained in step a) is added to the sample pad of a lateral flow device and
   a second member of the first binding pair associated to a marker capable of generating a colorimetric signal is immobilized on the conjugate pad of a lateral flow device
   and wherein
   the lateral flow device comprises a first area wherein a second member of the second binding pair has been immobilized, a second area wherein a second member of the third binding pair has been immobilized and a third area wherein a reagent capable of specifically binding to the second member of the first binding pair has been immobilized
   wherein the order of the first area and the second area in the lateral flow device is interchangeable
wherein the detection of a colorimetric signal in the first, second and third area of the lateral flow device indicates the presence of the target DNA sequence in the sample, or
wherein the absence of a colorimetric signal in the first area and the detection of a colorimetric signal in the second and third area of the lateral flow device indicates the absence of the target DNA sequence in the sample.

In a fourth aspect, the invention relates to a kit comprising a pair of primers capable of specifically amplifying a target DNA sequence wherein the first primer is labelled at the 5' end with a gold nanoparticle and the second primer is labelled at the 5' end with a first member of a binding pair, and the first member of a binding pair is bound to a second member of a binding pair conjugated to magnetic beads, and wherein said magnetic beads have a size lower than 2.8 µm and are separated from the region of the primer that recognizes the target DNA sequence by a polynucleotide spacer.

In a fifth aspect, the invention relates to a kit comprising:
(i) a pair of primers capable of specifically amplifying a target DNA sequence wherein the first primer is labelled at the 5' end with a gold nanoparticle and the second primer is labelled at the 5' end with a first member of a binding pair, and
(ii) a second member of a binding pair conjugated to magnetic beads.

In a sixth aspect, the invention relates to a kit comprising:
(i) a first pair of primers capable of specifically amplifying a target DNA sequence wherein the first primer of the first pair of primers is labelled at the 5' end with a first member of a first binding pair and the second primer of the first pair of primers is labelled at the 5' end with a first member of a second binding pair,
(ii) a second pair of primers capable of amplifying an endogenous amplification control DNA sequence wherein the first primer of the second pair of primers is labelled at the 5' end with said first member of said first binding pair and the second primer of the second pair of primers is labelled at the 5' end with a first member of a third binding pair,
   wherein said first members of the first, second and third binding pairs do not substantially cross-react with any second member of any different binding pair,
(iii) a recombinase,
(iv) a single-stranded DNA binding protein and
(v) a strand-displacing DNA polymerase.

In a further aspect, the invention relates to an *in vitro* method for diagnosing an infection by a pathogen in a subject comprising detecting the presence of a target DNA sequence from said pathogen in a biological sample of said subject by a method according to the invention.

In a further aspect, the invention relates to an oligonucleotide whose sequence is selected from the group consisting of SEQ ID NO: 1, SEQ ID NO: 2, SEQ ID NO: 3 and SEQ ID NO: 4.

### BRIEF DESCRIPTION OF THE FIGURES

**Figure 1****. (A)** Scheme of the procedure of the lateral flow assay of the invention. (B) Representation of the possible results depending on the sample assayed (positive or negative). Digoxig, digoxigenin.
**Figure 2****. (A)** Scheme of the enhanced lateral flow assay of the invention for isothermally amplified DNA detection. **(B)** Representation of the possible results depending on the sample assayed (positive or negative). Digoxig, digoxigenin.
**Figure 3****.** Optimization of primer concentration of *Leishmania* assay. +, positive sample (DNA of a dog with *Leishmania* infection); blanc, negative sample; ntc, negative template control.
**Figure 4****.** Isothermal amplification of the 18S ribosomal RNA gene used as endogenous control with primer 18s 1F and primer 18s 1R (18s 1F+1R). NTC: Negative Template Control; +: dog DNA.
**Figure 5****.** Results of the co-amplification of *Leishmania* parasite (K3R+K6F) and dog 18S rRNA gene as endogenous control visualized in an agarose gel electrophoresis (left) or in a lateral flow device (right). B: blank (DNA of a dog without *Leishmania* infection); + / A: positive sample (DNA of a dog with *Leishmania* infection).
**Figure 6****. Up:** Design of the masks used for the preparation of screen-printing electrodes. The different inks printed on a polyester sheet and detail of how these inks are placed in a single sensor. **Down:** Pictures of a polyester sheet with 45 printed sensors, detail of one single sensor and picture of the electrochemical set-up using a portable potentiostat.
**Figure 7****.** Scheme of the experimental procedure followed for the AuNPs synthesis and conjugation with: **(a)** anti-FITC antibodies and **(b)** thiol-modified primer. TEM image corresponds to a suspension of the 20-nm AuNPs obtained.
**Figure 8****.** Scheme of the experimental procedure for isothermal amplification using primers labelled with AuNPs and biotin (step 1), magnetic capturing of the isothermally amplified product (step 2) and electrocatalytic detection (step 3).
**Figure 9****.** Scheme of the experimental procedure for the detection of isothermally amplified DNA using primers labelled with AuNPs and MBs.
**Figure 10****.** Lateral flow results of the specificity assay with other pathogens. NTC: negative template control.
**Figure 11****.** Lateral flow results of the specificity assay with different isothermal reagents.
**Figure 12****.** Results of parasitemia of different samples obtained by isothermal amplification and agarose gel electrophoresis detection.
**Figure 13****.** Limit of detection using commercial lateral flow **(A)** or agarose gel electrophoresis **(B).**
**Figure 14****.** Pictures of lateral flow assay strips after the assay performed for isothermally amplified *Leishmania* DNA at different dilution factors, containing the endogenous control. The direct and the enhanced assays are also compared. CL: migration control; TL2: endogenous control; TL1: target detection.
**Figure 15****. (A)** Pictures of lateral flow assay strips after the assay performed for isothermally amplified *Leishmania* DNA from samples with different quantities of parasites, containing the endogenous control. **(B)** Corresponding intensity values (analytical signal) obtained with the strip reader. **(C)** Relationship between the parasite concentration and the analytical signal. CL: migration control; TL2: endogenous control; TL1: target detection; TBST: Tris buffered saline and Tween 20.
**Figure 16****.** Diagram for the zeta potential as a distribution versus total counts for a dispersion of AuNPs before (a curve) and after (b curve) the conjugation with the primer and for amplified DNA using the AuNP-labelled primer for a positive (d curve) and a blank (c curve) sample.
**Figure 17****.** Comparison of the analytical signals obtained for different "positive" and "blank" samples after DNA amplification using AuNP-labelled primers.
**Figure 18****.** Analytical signals electrochemically detected obtained for different isothermal amplifications performed several weeks after the preparation of the conjugate of AuNP/primer.
**Figure 19****.** Analytical signals electrochemically detected obtained for different isothermal amplifications performed using different sized MBs and primers with and without spacer.

### DETAILED DESCRIPTION OF THE INVENTION

The inventors of the present invention have discovered that the amplification of a target DNA by a recombinase-polymerase isothermal amplification and the detection of the amplified products by lateral flow or electrochemistry allows diagnosing infectious diseases in a more rapid, sensitive and specific way.

Particularly, the inventors propose two assays for *Leishmania* kinetoplast identification that comprise a first step of amplification of the target DNA by a recombinase-polymerase isothermal amplification (RPA) technique. This technique is rapid, cost effective, easy-to-use and more tolerant to inhibitory components from a crude sample compared to PCR. It is a powerful tool for POC diagnostic since it does not have the additional complexity of PCR thermal cycling steps. The amplification step involves the use of labelled primers to generate a double labelled product. The labels of said primers are used in the subsequent step for the detection of the amplified products. An advantage of said labelled primers is that they avoid the use of internal probes for the detection of the amplified products. Probes can be difficult to design when the amplicon has a high polymorphism and the methods of the invention overcome this disadvantage.

The first assay involves the amplification of *Leishmania* kinetoplast by RPA with labelled primers combined with the advantages of magnetic purification/preconcentration and the use of gold nanoparticle (AuNPs) tags for the quantitative electrochemical detection of the amplified products. The integration of nanoparticles in one of the strands of the amplicon allows a stable, sensitive and low cost detection.

The second assay involves the co-amplification of *Leishmania* kinetoplast and dog 18S rRNA gene as endogenous control by RPA and the optical detection of the amplified products using a universal lateral flow device or an enhanced lateral-flow assay using secondary antibodies. This assay incorporates an endogenous control that serves as a positive control of the correct recombinase-polymerase amplification process and avoids the generation of artefacts that were detected in no-template controls and samples of very low target copy number. This is achieved by using labelled primers to obtain a double labelled amplicon which is simpler than a method that requires the use of probes. Additionally, the enhanced lateral-flow assay proposed by the inventors is a versatile and universal methodology that can be applied for any lateral flow design and has the advantage that the primary antibody against the amplified product does not need to be directly labelled. A secondary standard antibody (e.g. anti-goat, anti-chicken) is labelled, with clear advantages in terms of technology cost.

### FIRST METHOD OF THE INVENTION

In a first aspect, the invention relates to an *in vitro* method (hereinafter referred to as "first method of the invention") for detecting a target DNA sequence in a sample comprising the following steps:
a) submitting the sample DNA to a recombinase-polymerase isothermal nucleic acid amplification in the presence of a pair of primers capable of specifically amplifying the target DNA sequence wherein the first primer is labelled at the 5' end with a gold nanoparticle and the second primer is labelled at the 5' end with a first member of a binding pair, and
b) measuring the electrochemical signal produced by the gold nanoparticles forming part of the amplification products obtained in step a) using a screen-printed carbon electrode after capture of the amplification products on the electrode using magnetic beads conjugated to a second member of a binding pair,
wherein the detection of an electrochemical signal superior to the background signal indicates the presence of the target DNA sequence in the sample.

The target DNA sequence can be any DNA sequence. This includes, without limitation, genomic DNA (nuclear DNA, mitochondrial DNA, chloroplast DNA, etc.), and plasmid DNA. An expert in the field can access any target DNA sequence through public databases, for example GenBank. The target DNA sequence can be a nucleic acid from an animal (e.g. human), plant, fungal (e.g. yeast), protozoan, bacterial, or viral species. For example, the target DNA sequence can be present in the genome of an organism of interest. In a particular embodiment the target DNA sequence is specific for the organism of interest, i.e. the target nucleic acid is not found in other organisms or not found in organisms similar to the organism of interest. In a preferred embodiment the target DNA sequence is from a pathogen, particularly is from an infectious organism.

In an embodiment the target DNA sequence is a sequence from bacteria, e.g., Gram-positive or Gram-negative bacteria. Exemplary bacterial species include, without limitation, *Acinetobacter sp.* strain ATCC 5459, *Acinetobacter calcoaceticus, Aerococcus viridans, Bacteroides fragilis, Bordetella pertussis, Bordetella parapertussis, Campylobacter jejuni, Clostridium difficile, Clostridium perfringens, Corynebacterium sp., Chlamydia pneumoniae, Chlamydia trachomatis, Citrobacter freundii, Enterobacter aerogenes, Enterococcus gallinarum, Enterococcus faecium, Enterobacter faecalis* (e.g., ATCC 29212), *Escherichia coli* (e.g., ATCC 25927), *Gardnerella vaginalis, Helicobacter pylori, Haemophilus influenzae* (e.g., ATCC 49247), *Klebsiella pneumoniae, Legionella pneumophila* (e.g., ATCC 33495), *Listeria monocytogenes* (e.g., ATCC 7648), *Micrococcus sp.* strain ATCC 14396, *Moraxella catarrhalis, Mycobacterium kansasii, Mycobacterium gordonae, Mycobacterium fortuitum, Mycoplasma pneumoniae, Mycoplasma hominis, Neisseria meningitis* (e.g., ATCC 6250), *Neisseria gonorrhoeae, Oligella urethralis, Pasteurella multocida, Pseudomonas aeruginosa* (e.g., ATCC 10145), *Propionibacterium acnes, Proteus mirabilis, Proteus vulgaris, Salmonella sp.* strain ATCC 31194, *Salmonella typhimurium, Serratia marcescens* (e.g., ATCC 8101), *Staphylococcus aureus* (e.g., ATCC 25923), *Staphylococcus epidermidis* (e.g., ATCC 12228), *Staphylococcus lugdunensis, Staphylococcus saprophyticus, Streptococcus pneumoniae* (e.g., ATCC 49619), *Streptococcus pyogenes, Streptococcus agalactiae* (e.g., ATCC 13813), *Treponema pallidum, Viridans* group streptococci (e.g., ATCC 10556), *Bacillus anthracis, Bacillus cereus, Francisella philomiragia* (GA01-2810), *Francisella tularensis* (LVSB), *Yersinia pseudotuberculosis* (PB 11+), *Yersinia enterocolitica,* 0:9 serotype, and *Yersinia pestis* (P14-). In some embodiments, the target DNA sequence is from a species of a bacterial genus selected from *Acinetobacter, Aerococcus, Bacteroides, Bordetella, Campylobacter, Clostridium, Corynebacterium, Chlamydia, Citrobacter, Enterobacter, Enterococcus, Escherichia, Helicobacter, Haemophilus, Klebsiella, Legionella, Listeria, Micrococcus, Mobilincus, Moraxella, Mycobacterium, Mycoplasma, Neisseria, Oligella, Pasteurella, Prevotella, Porphyromonas, Pseudomonas, Propionibacterium, Proteus, Salmonella, Serratia, Staphylococcus, Streptococcus, Treponema, Bacillus, Francisella,* and *Yersinia.* In another embodiment, the target DNA sequence is from a species of a bacterial genus selected from the group consisting of *Ehrlichia, Rickettsia, Bartonella, Borrelia, Coxiella, Mycoplasma, Chlamydophila* and *Anaplasma.* Particularly, the target DNA sequence is from a bacterial species selected from the group consisting of *Anaplasma platys, Anaplasma phagocytophilum, Ehrlichia canis, Ehrlichia chafensis, Borrelia burgdorferi, Coxiella burnetii, Rickettsia rickettsii, Rickettsia conorii, Rickettsia felis, Rickettsia helvetica, Rickettsia massielae, Rickettsia monacensis, Rickettsia typhi, Bartonella henselae, Bartonella clarridgeiae, Bartonella rochalimae, Bartonella taylorii, Bartonella birtlesii, Bartonella vinsonii, Mycoplasma ovis, Mycoplasma suis, Mycoplasma haemofelis, Mycoplasma haemocanis, Candidatus* Mycoplasma haematoparvum, *Candidatus* Mycoplasma hamominutum, *Candidatus* Mycoplasma turicensis and *Chlamydophila psittacii.* In a preferred embodiment, the target DNA sequence is from a species of a bacterial genus selected from the group consisting of *Ehrlichia* and *Anaplasma.*

In another embodiment, the target DNA sequence is a viral DNA sequence. For example, without limitation, the viral DNA can be from human immunodeficiency virus (HIV), influenza virus, dengue virus, herpes simplex virus, varicella-zoster virus, Epstein-barr virus, cytomegalovirus, papillomavirus, BK virus, JC virus, smallpox, hepatitis B virus, parvovirus, astrovirus, Norwalk virus, coxsackievirus, hepatitis A virus, poliovirus, rhinovirus, severe acute respiratory syndrome virus, hepatitis C virus, yellow fever virus, West Nile virus, rubella virus, hepatitis E virus, Lassa virus, haemorrhagic fever virus, Ebola virus, Marburg virus, measles virus, mumps virus, parainfluenza virus, respiratory syncytial virus, metapneumovirus, Hendra virus, Nipah virus, rabies virus, hepatitis D virus, rotavirus, orbivirus, coltivirus or Banna virus. In another embodiment, the target DNA sequence is from a virus selected from the group consisting of canine distemper virus, canine herpesvirus, feline coronavirus, Beak and feather disease virus (BFDV), polyomavirus and herpesvirus.

In another embodiment, the target DNA sequence is a protozoan DNA sequence. For example, the protozoan DNA sequence can be, without limitation, a DNA sequence from *Babesia divergens, Babesia bigemina, Babesia equi, Babesia microti, Babesia duncani, Plasmodium spp., Leishmania spp., Trypanosoma brucei gambiense, Trypanosoma brucei rhodesiense, Trypanosoma cruzi, Entamoeba spp., Toxoplasma spp., Trichomonas vaginalis,* or *Giardia duodenalis.* In another embodiment, the target DNA sequence is from a species of a protozoan genus selected from the group consisting of *Toxoplasma, Hepatozoon, Neospora, Babesia* and *Theileria.* Preferably, the target DNA sequence is from a protozoan species selected from the group consisting of *Toxoplasma gondii, Hepatozoon canis, Hepatozoon felis, Neospora caninum, Babesia gibsoni, Babesia canis, Babesia canis canis, Babesia canis vogeli, Babesia microti, Babesia equi, Babesia rossi* and *Theileria annae.* In a preferred embodiment, the target DNA sequence is from a species of a protozoan genus selected from the group consisting of *Babesia* and *Theileria.* In a more preferred embodiment, the target DNA sequence is from an organism of the *Kinetoplastea* class.

The *Kinetoplastea* class is a group of single-cell flagellate protozoa, including a number of parasites responsible for diseases in humans and other animals, as well as various forms found in soil and aquatic environments. They are members of the phylum *Euglenozoa* and their major distinguishing feature is the presence of a kinetoplast (called kDNA), a dense DNA-containing granule located within the single mitochondrion that contains the mitochondrial genome. The kinetoplast is found at the base of a cell's flagella and is associated to the flagellum basal body by a cytoskeletal structure. The kinetoplast is easily visible in samples stained with DAPI or by the use of FISH with BrdU. *Kinetoplastea* class includes, without limitation, species of *Trypanosoma* and *Leishmania* genus.

The assay of the present invention targets a conserved region of the *Leishmania* kinetoplast minicircle DNA that is present in about 10,000 copies for parasite. Therefore, in a preferred embodiment the target DNA sequence is a sequence of the kinetoplast, preferably the sequence of the kinetoplast of *Leishmania infantum.* The sequence of the kinetoplast from several *Leishmania infantum* isolates may be found on GenBank. Exemplary DNA sequences from Leishmania infantum kinetoplast DNA are, without limitation, sequences with GenBank accession numbers Z35500.1 (dated 9 September 2004), AF169133.1 (dated 1 December 2000), AF103735.1 (dated 2 July 1999), AF103741.1 (dated 2 July 1999), AF169131.1 (dated 1 December 2000), AF169140.1 (dated 1 December 2000), Z35274.1 (dated 15 July 1994), Z35292.1 (dated 18 July 1994), AF184044.1 (dated 30 November 2000), AF190475.1 (dated 30 November 2000), Z35270.1 (dated 15 July 1994), EU437407.1 (dated 31 January 2010), AF188701.1 (dated 30 November 2000), AF190883.1 (dated 27 October 1999), EU437405.1 (dated 31 January 2010), EU437403.1 (dated 31 January 2010), EU437406.1 (dated 31 January 2010), Z35269.1 (dated 15 July 1994), Z35501.1 (dated 9 September 2004), EU437404.1 (dated 31 January 2010), AF190476.1 (dated 30 November 2000), AF190882.1 (dated 27 October 1999), Z35271.1 (dated 15 July 1994), Z35273.1 (dated 15 July 1994) and Z35272.1 (dated 15 July 1994).

In another embodiment, the target DNA sequence is a DNA sequence from a parasite. For example, the parasite DNA sequence can be, without limitation, a DNA sequence from *Acanthamoeba, Balamuthia mandrillaris, Babesia Balantidium coli, Blastocystis, Cryptosporidium, Dientamoeba fragilis, Entamoeba histolytica, Giardia lamblia, Giardia duodenalis, Isospora belli, Leishmania, Naegleria fowleri, Plasmodium falciparum, Plasmodium vivax, Plasmodium ovale, Plasmodium malariae, Plasmodium knowlesi, Rhinosporidium seeberi, Sarcocystis bovihominis, Sarcocystis suihominis, Toxoplasma gondii, Trichomonas vaginalis, Trypanosoma brucei, Trypanosoma cruzi, Taenia multiceps, Diphyllobothrium latum, Echinococcus granulosus, Echinococcus multilocularis, Echinococcus vogeli, Echinococcus oligarthrus, Hymenolepis nana, Hymenolepis diminuta, Taenia saginata, Taenia solium, Bertiella mucronata, Bertiella studeri, Spirometra erinaceieuropaei, Clonorchis sinensis, Clonorchis viverrini, Dicrocoelium dendriticum, Fasciola hepatica, Fasciola gigantica, Fasciolopsis buski, Gnathostoma spinigerum, Gnathostoma hispidum, Metagonimus yokogawai, Opisthorchis viverrini, Opisthorchis felineus, Clonorchis sinensis, Paragonimus westermani, Paragonimus africanus, Paragonimus caliensis, Paragonimus kellicotti, Paragonimus skrjabini, Paragonimus uterobilateralis, Schistosoma sp., Schistosoma mansoni, Schistosoma haematobium, Schistosoma japonicum, Schistosoma mekongi, Echinostoma echinatum, Trichobilharzia regenti, Ancylostoma duodenale, Angiostrongylus costaricensis, Anisakis, Ascaris sp., Ascaris lumbricoides, Baylisascaris procyonis, Brugia malayi, Brugia timori, Dioctophyme renale, Dracunculus medinensis, Enterobius vermicularis, Enterobius gregorii, Halicephalobus gingivalis, Loa loa filaria, Mansonella streptocerca, Onchocerca volvulus, Strongyloides stercoralis, Thelazia californiensis, Thelazia callipaeda, Toxocara canis, Toxocara cati, Trichinella spiralis, Trichinella britovi, Trichinella nelsoni, Trichinella nativa, Trichuris trichiura, Trichuris vulpis, Wuchereria bancrofti, Archiacanthocephala, Moniliformis moniliformis, Linguatula serrata, Tunga penetrans, Dermatobia hominis, Cimex lectularius, Pediculus humanus, Pediculus humanus corporis, Pthirus pubis, Demodex folliculorum, Demodex brevis, Demodex canis, Sarcoptes scabiei, Cochliomyia hominivorax,* or *Pulex irritans.* In another embodiment, the target DNA sequence is from a species of a parasite genus selected from the group consisting of *Angiostrongylus, Demodex, Thelazia, Dirofilaria,* and *Acantocheilonema.* Particularly, the target DNA sequence is from a parasite species selected from the group consisting of *Angiostrongylus vasorum, Demodex canis, Demodex cornei, Demodex injai, Demodex cati, Demodex gatoi, Dirofilaria immitis, Dirofilaria repens, Acantocheilonema dracunculoides,* and *Thelazia callipaeda.*

In a preferred embodiment, the target DNA sequence is a sequence from *Leishmania,* preferably a sequence selected from a species of the group consisting of *L. aethiopica, L. amazonensis, L. arabica, L. archibaldi, L. aristedes, L. braziliensis, L. chagasi, L. colombiensis, L. deanei, L. donovani, L. enriettii, L. equatorensis, L. forattinii, L. garnhami, L. gerbili, L. guyanensis, L. herreri, L. hertigi, L. infantum, L. killicki, L. lainsoni, L. major, L. mexicana, L. naiffi, L. panamensis, L. peruviana, L. pifanoi, L. shawi, L. turanica, L. tropica* and *L. venezuelensis;* more preferably selected from a species of the group consisting of *L. aethiopica, L. braziliensis, L. chagasi, L. donovani, L. infantum, L. major, L. mexicana* and *L. tropica.* In a more preferred embodiment, the target DNA sequence is from *Leishmania infantum.*

In another embodiment, the target DNA sequence is a fungal (e.g. yeast) DNA sequence. For example, the fungal DNA sequence can be found, without limitation, in *Candida spp.* (e.g. *Candida albicans), Aspergillus* (e.g. *Aspergillus fumigatus, Aspergillus flavus, Aspergillus clavatus*), *Cryptococcus* (e.g. *Cryptococcus neoformans, Cryptococcus laurentii, Cryptococcus albidus, Cryptococcus gattii*), *Histoplasma capsulatum, Pneumocystis carinii* or *Stachybotrys chartarum.*

In another embodiment, the target DNA sequence is a mammalian DNA sequence (e.g. human, cows, horses, pigs, sheep, goats, dogs, cats, rodents, etc.). For example, the mammalian DNA sequence can be a sequence found in circulating tumour cells, epithelial cells, or fibroblasts.

The term "sample", as used herein, includes a biological sample (e.g. blood, bone marrow, urine, saliva, sputum, lymph, plasma, ejaculate, lung aspirate, cerebrospinal fluid and biopsy without limitation) and a sample from an environmental source. Biological samples include, without limitation, animal or human samples, liquid and solid food and feed products (dairy items, vegetables, meat, etc.). Preferred biological samples include, without limitation, any biological fluid, cell, tissue, organ or portion thereof that contains DNA. Preferably, the biological sample is selected from lesional swab, throat swab, nasal swab, vaginal swab and rectal swab. A biological sample can include a neoplastic cell. Said sample can be from any organism and includes, but is not restricted to, bacteria, fungi, viruses, plants, animals, e.g. human beings, non-human primates, reptiles, insects, birds, worms, fish, mammals, domestic and farm animals (cows, horses, pigs, sheep, goats, dogs, cats, rodents, etc.). In a particular embodiment the sample is from an organism different than the organism from which the target DNA sequence originates. In an embodiment, the sample is from a mammal selected from dogs, cats, rodents, preferably rats and hamsters. In a preferred embodiment the sample is from a dog. In another embodiment the sample is from a human being. Environmental samples include, without limitation, surface matter, soil, water and industrial samples, as well as samples obtained from food and dairy processing instruments. The sample being analysed can be derived from a single source (e.g. a single organism, tissue, cell, etc.) or be a pool of DNA from a plurality of organisms, tissues or cells. Said sample can be obtained by conventional methods. Extraction of DNA from the sample may be carried out by methods well known by the person skilled in the art.

In a first step, the method for detecting a target DNA sequence according to the first method of the invention involves submitting the sample DNA to recombinase-polymerase isothermal nucleic acid amplification.

The term "recombinase-polymerase isothermal nucleic acid amplification" (also known as RPA), as used herein, refers to the isothermal amplification method operating best at constant temperatures between 37-42°C that employs three core enzymes: a recombinase, a single-stranded DNA-binding protein (SSB) and a strand-displacing polymerase. The RPA method comprises the following steps: (i) first, a recombinase is contacted with a first and a second nucleic acid primers to form a first and a second nucleoprotein primers; (ii) second, the first and second nucleoprotein primers are contacted to a double stranded target sequence to form a first double stranded structure at a first portion of said first strand and form a double stranded structure at a second portion of said second strand so the 3' ends of said first nucleic acid primer and said second nucleic acid primer are oriented towards each other on a given template DNA molecule; and the single-stranded DNA binding protein binds to displaced strands of DNA to prevent the primers from being displaced; (iii) third, the 3' end of said first and second nucleoprotein primers are extended by a strand-displacing DNA polymerase to generate first and second double stranded nucleic acids, and first and second displaced strands of nucleic acids, and (iv) finally, the second and third steps are repeated until a desired degree of amplification is reached. No thermal or chemical melting is required to initiate amplification and the reaction progresses typically within 5-10 minutes. A description of the RPA technique may be found on European patent EP 1 759 012 B1.

Suitable conditions for carrying out the RPA can be found on the materials and methods section and can be determined by the person skilled in the art. In a preferred embodiment, the RPA is carried out at a temperature of 37°C.

The RPA of the first method of the invention is performed in the presence of a pair of primers capable of specifically amplifying the target DNA sequence wherein the first primer is labelled at the 5' end with a gold nanoparticle and the second primer is labelled at the 5' end with a first member of a binding pair.

The term "primer" as used herein, refers to a short strand of nucleic acid that is complementary to a sequence in another nucleic acid and serves as a starting point for DNA synthesis. Preferably, the primer has at least 5, at least 6, at least 7, at least 8, at least 9, at least 10, at least 11, at least 12, at least 13, at least 14, at least 15, at least 16, at least 18, at least 20, at least 25, at least 30 or more bases long. More preferably, the optimum length is between 30-35 bases long.

The expression "capable of specifically amplifying", as used herein, means that the primer is designed to be complementary to the target DNA sequence to be amplified, (i.e. refers to the base pairing that allows the formation of a duplex between the primer and its complementary sequence in a DNA molecule). Complementary nucleotides are, generally, A and T (or A and U), or C and G. Two single-stranded DNA molecules are said to be substantially complementary when the nucleotides of one strand, optimally aligned and compared and with appropriate nucleotide insertions or deletions, pair with about 60% of the other strand, at least 70%, at least 80%, at least 85%, usually at least about 90% to about 95%, and even about 98% to about 100%. The degree of identity between two nucleotide regions is determined using algorithms implemented in a computer and methods which are widely known by the persons skilled in the art. The identity between two nucleotide sequences is preferably determined using the BLASTN algorithm (BLAST Manual, Altschul, S. et al., NCBI NLM NIH Bethesda, Md. 20894, Altschul, S., et al., J., 1990, Mol. Biol. 215:403-410). The primers hybridize to the target DNA sequence. "Hybridization" refers to the process in which two single-stranded polynucleotides bind non-covalently to form a stable double-stranded polynucleotide.

The expression "specifically", when referred to the amplification of a target DNA sequence by a pair of primers, means that the primers are capable of hybridizing to an amplify the target DNA sequence but they are not capable of hybridizing and amplifying other sequences different from the target DNA sequence, thus allowing a specific detection of said sequence.

Primers suitable for the methods of the invention are primers without long tracks of guanines at the first 3-5 nucleotides of the 5' end and without unusual sequence elements such as homopolymers. The preferred primers have GC content between 30% and 70%. In order to improve performance, primers having cytidines at the 5' end and guanines and cytidines at the last 3 nucleotides of the 3' end are preferred. The amplicon length preferably does not exceed from 500 bp. The ideal amplicon length is between 100-200 bp.

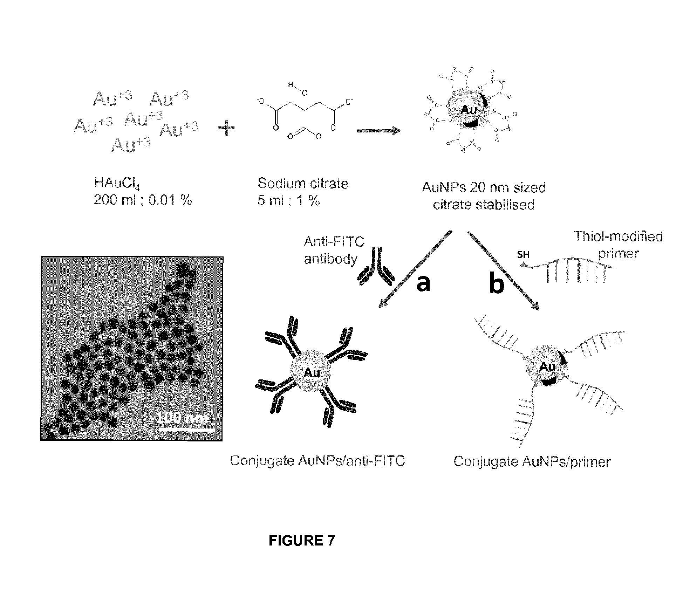

The first primer of the first method of the invention is labelled at the 5' end with a gold nanoparticle. The term "5' end", as used herein, designates the end of a nucleotide strand that has the fifth carbon in the sugar-ring of the deoxyribose at its terminus. The expression "gold nanoparticle" or "AuNP", as used herein, refers to an ultrafine particle between 2 and 200 nm in size that is formed by gold atoms. In a preferred embodiment gold nanoparticles have 20 nm in size. Gold nanoparticles suitable for the present invention are gold nanoparticles obtained by a simple method of synthesis, having a narrow size distribution, capable of being easily conjugated to antibodies and/or ssDNA and having electro(catalytic) activity. Generally, gold nanoparticles are produced in a liquid by reduction of chloroauric acid. Different methods are known by the person skilled in the art to manufacture gold nanoparticles. In a preferred embodiment the method used is Turkevich's method (Turkevich et al. Discuss. Faraday Soc. 1951, 11:55-75) that is disclosed in section 3.2 of the experimental part of the present description. In order to label the 5' end of the first primer with a gold nanoparticle said primer has to be previously modified with thiol groups and then the AuNPs/primer conjugate can be obtained. A method for modifying the first primer with thiol groups and conjugating it with AuNPs is disclosed in section 3.3 of Examples. Figure 7 shows a scheme of the synthesis of AuNPs and their conjugation with antibodies and primers.

The second primer of the first method of the invention is labelled at the 5' end with a first member of a binding pair. In an embodiment, the second primer used in step a) of the first method of the invention is not bound to the second member of the binding pair conjugated to magnetic beads. In another embodiment, the second primer used in step a) of the first method of the invention is bound to the second member of the binding pair conjugated to magnetic beads.

The term "binding pair", in the context of the first method of the invention, refers to a pair formed by a first member and a second member and includes any of the class of immune-type binding pairs, such as antigen/antibody (for example, digoxigenin/anti-digoxigenin antibody) or hapten/anti-hapten systems; and also any of the class of non-immune-type binding pairs which include systems wherein the two components share a natural affinity for each other but are not antibodies, such as biotin/avidin, biotin/streptavidin, folic acid/folate binding protein and protein A or G/immunoglobulins. The second primer is labelled with a first member of a binding pair by methods well known in the art.

In an embodiment, the first member of a binding pair is an antigen or hapten. The term "antigen", as used herein, refers to any substance which provokes an adaptative immune response. The term "hapten", as used herein, refers to a small molecule that changes the structure of an antigenic epitope and that has to be attached to a large carrier molecule such as a protein to induce an immune response.

In another embodiment, the first member of a binding pair is biotin.

In an embodiment, the second member of the binding pair is an antibody specific for the antigen or hapten of the first member of the binding pair. The term "specific", in the context of the antibody used in the first method of the invention, means that said antibody is capable of recognizing a particular antigen and no other different antigens.

In another embodiment, the second member of a binding pair is streptavidin.

The second member of the binding pair can complex with the first member of the binding pair. Said second member of the binding pair is conjugated to magnetic beads.

The term "magnetic beads", as used herein, refers to magnetic particles having a size between 0.5 to 500 µm that are uniform particles having magnetic properties when placed in a magnetic field and with no residual magnetism once removed from the magnetic field. Such particles commonly contain magnetic elements such as iron, nickel and cobalt and their chemical compounds. The magnetic beads can be paramagnetic or super-paramagnetic. In an embodiment, magnetic beads are super-paramagnetic. Magnetic beads are preferably spherical. In some embodiments, magnetic beads having a true spherical shape and defined surface chemistry are used to minimize chemical agglutination and non-specific binding. In a preferred embodiment, magnetic beads have a size lower than 2.8 µm, preferably lower than 2.5 µm, more preferably lower than 2.0 µm, even more preferably lower than 1.5 µm, even more preferably lower than 1.0 µm. In an embodiment the magnetic beads have a size of 1.0 µm.

Magnetic beads and methods for their preparation are well-known and they are widely available commercially, with or without functional groups capable of binding to affinity molecules. Suitable magnetic beads are commercially available such as from Dynal Inc. (Lake Success, NY); PerSeptive Diagnostics, Inc. (Cambridge, MA); Invitrogen Corp. (Carlsbad, CA); Cortex Biochem Inc. (San Leandro, CA); and Bangs Laboratories (Fishers, IN). In particular embodiments, magnetic particles are MyOne™ Dynabeads(R) magnetic beads (Dynal Inc.).

The term "conjugated", as used herein, means that the second member of a biding pair is coupled to the surface of the magnetic bead. Said modified magnetic beads may be manufactured by methods well known by the person skilled in the art or may be commercially available.

The first member of the binding pair may be bound to the second member of a binding pair before or after the amplification takes place.

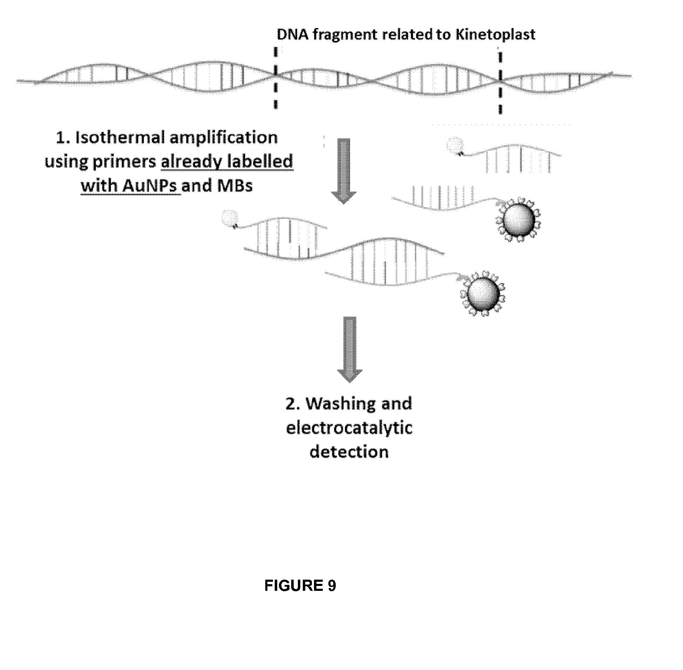

If the first member of the binding pair is bound to the second member of the binding pair before the amplification takes place, this means that the second primer is modified with magnetic beads. A method for obtaining a primer modified with magnetic beads is described in section 3.6 of Material and methods and Figure 9. The experimental section shows that if the second primer is modified with magnetic beads said magnetic beads must have a size lower than 2.8 µm and must separate from the region of the primer that recognizes the target DNA sequence by a polynucleotide spacer. Preferably, when the second primer is modified with magnetic beads the second member of the binding pair is not an antibody. This design allows reducing time and cost of the analysis of the amplified product. Therefore, in an embodiment the second primer labelled at the 5' end with a first member of a binding pair is bound to the second member of the binding pair conjugated to magnetic beads before step a) takes place, and wherein said magnetic beads have a size lower than 2.8 µm and are separated from the region of the primer that recognizes the target DNA sequence by a polynucleotide spacer.

The expression "polynucleotide spacer", as used herein, refers to a nucleotide sequence that links the region of the primer that recognizes the target DNA sequence and the magnetic bead and which acts as a hinge region, providing space between both elements and assuring that the amplification process is not affected by the presence of the magnetic bead. The polynucleotide spacer may be of any length that allows accomplishing said functions. In a preferred embodiment, the polynucleotide spacer is a nucleotide sequence with a length of 10-30 nucleotides, preferably 15-20 nucleotides, more preferably has a length of 15 nucleotides. In an embodiment, the polynucleotide spacer is the sequence ATATATATATATATA (SEQ ID NO: 5).

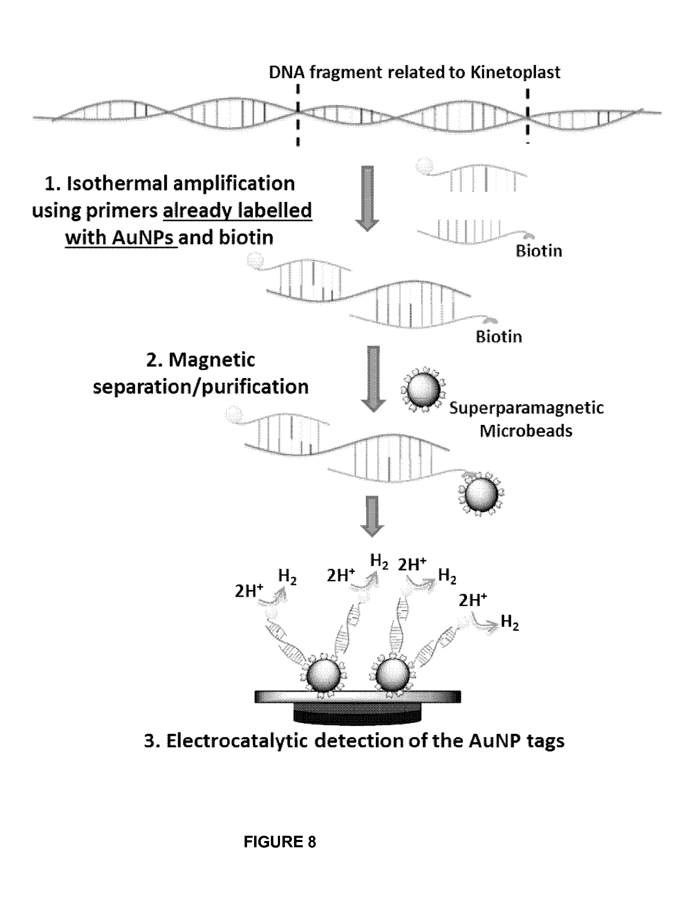

If the first member of the binding pair is bound to the second member of the binding pair after the amplification takes place, this means that the amplified product labelled with a first member of a binding pair is captured after amplification by a second member of the binding pair. Therefore, in an embodiment the second member of the binding pair conjugated to magnetic beads is added after step a). This embodiment is described in section 3.5 of Material and methods and Figure 8.

In an embodiment, the method for detecting a target DNA sequence according to the first method of the invention involves capturing the amplification products obtained in step a) in the presence of a magnetic bead conjugated to a second member of a binding pair before step b) takes place.

The expression "amplification product", as used herein, refers to a double strand amplicon wherein the first strand is labelled at the 5' end with a gold nanoparticle and the second strand is labelled at the 5' end with a first member of a binding pair.

The expression "in the presence of a magnetic bead", as used herein, means that a magnetic bead is associated to the second strand of the amplified product either because the second member of the binding pair conjugated to magnetic beads is added after step a) or because the second primer labelled at the 5' end with a first member of a binding pair is bound to the second member of the binding pair conjugated to magnetic beads before step a) takes place.

The presence of a magnetic bead associated to the amplification product is necessary to recover said product from the reaction mixture. This capture is performed by magnetic separation of the products associated to the magnetic beads.

When the second primer labelled at the 5' end with a first member of a binding pair is bound to the second member of the binding pair conjugated to magnetic beads before step a) takes place, the product obtained in step a) is washed for removing the excess of primers before step b) takes place. Therefore, in an embodiment, the product obtained in step a) is washed for removing the excess of primers before step b) takes place.

In a second step (step b), the method for detecting a target DNA sequence according to the first method of the invention involves measuring the electrochemical signal produced by the gold nanoparticles forming part of the amplification products obtained in step a) using a screen-printed carbon electrode after capture of the amplification products on the electrode using magnetic beads conjugated to a second member of a binding pair.

In this final step the captured magnetic beads associated to the amplification product are placed on the surface of a screen-printed carbon electrode that has a magnet attached to the reverse side of the surface. Gold nanoparticles are detected taking advantage of their electroactive properties. In a preferred embodiment the electrocatalytic activity of gold nanoparticles towards the electroreduction of hydrogen ions in an acidic media is approached for their sensitive detection. In order to measure the electrochemical signal produced by the captured products, a solution of HCl is added, and a potential is applied to reduce H⁺ ions to H₂ due to the catalytic effect of the gold nanoparticles. Quantitative measurements may be performed according to the disclosure of section 3.7 of Materials and methods.

The expression "electrochemical signal", as used herein, refers to the absolute value of the current registered at 100 seconds when a potential of -1.00 V was applied in chronoamperometric mode.

The expression "biosensor", as used herein, refers to an analytical device used for the detection of the amplified product that combines a biological component (the sensitive biological element that recognizes the amplified product under study, i.e. the second member of a binding pair conjugated to magnetic beads) with a physicochemical detector (electrochemical transducer) that transforms the signal resulting from the interaction of the amplified product with the biological element into another signal more easily measured and quantified. The biosensor of the present invention must capture the magnetic beads and therefore, it is made from a metallic material.

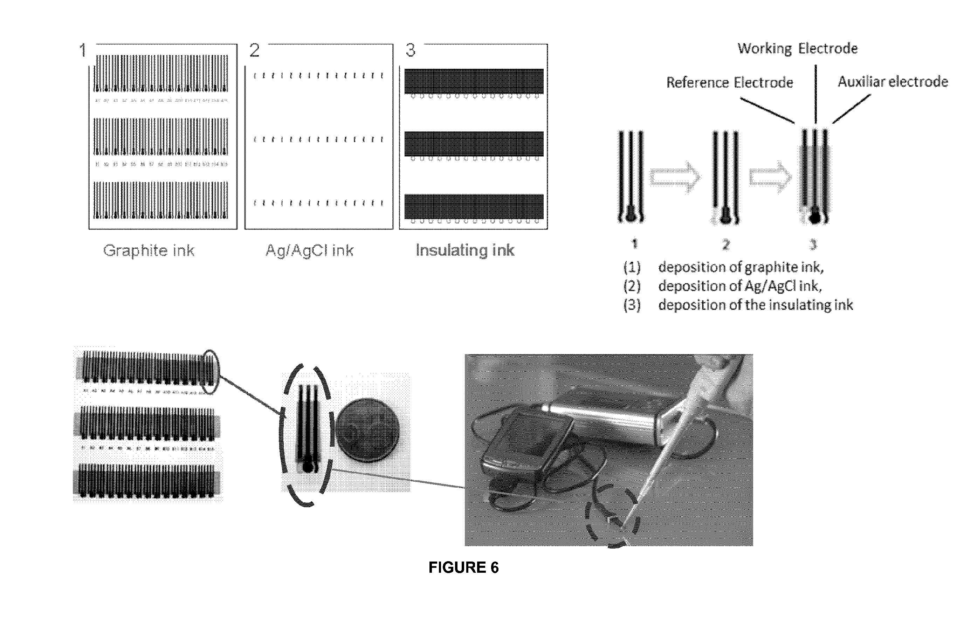

The electrochemical transducer (or electrode) is a screen-printed carbon electrode (SPCE) which includes a carbon working electrode, a carbon counter electrode and a silver/silver chloride reference electrode. SPCE and their manufacture methods are well known in the state of the art. A method for manufacturing SPCEs is described in section 3.1 of Material and methods wherein disposable SPCEs that require the use of only 25 µl of sample have been manufactured. Figure 6 shows details of this procedure. In a preferred embodiment the screen-printed carbon electrode is printed on a polyester sheet.

In the first method of the invention the detection of an electrochemical signal superior to the background signal indicates the presence of the target DNA sequence in the sample. The background signal corresponds to the absolute value of the current (recorded at 100 seconds) by the H⁺ ions reduction in the absence of gold nanoparticles.

The electrochemical signal is proportional to the quantity of gold nanoparticles and, consequently, to the concentration of the amplified product.

### SECOND METHOD OF THE INVENTION

In a second aspect, the invention relates to an *in vitro* method (hereinafter referred to as "second method of the invention") for detecting a target DNA sequence in a sample comprising the following steps:
a) submitting the sample DNA to a recombinase-polymerase isothermal nucleic acid amplification in the presence of a pair of primers capable of specifically amplifying the target DNA sequence wherein the first primer is labelled at the 5' end with a first member of a first binding pair and the second primer is labelled at the 5' end with a first member of a second binding pair, and
   wherein said first members of the first and second binding pairs do not substantially cross-react with any second member of any different binding pair, and
b) measuring the electrochemical signal produced by the gold nanoparticles forming part of the amplification products obtained in step a) using a screen-printed carbon electrode after the first member of a first binding pair is bound to a second member of a first binding pair conjugated to a gold nanoparticle and after capture of the amplification products on the electrode using magnetic beads conjugated to a second member of a second binding pair
wherein the detection of an electrochemical signal superior to the background signal indicates the presence of the target DNA sequence in the sample.

The terms disclosed in steps a) and b) of the second method of the invention have been defined previously in the context of the first method of the invention.

The RPA of step a) of the second method of the invention is performed in the presence of a pair of primers capable of specifically amplifying the target DNA sequence wherein the first primer is labelled at the 5' end with a first member of a first binding pair and the second primer is labelled at the 5' end with a first member of a second binding pair and wherein said first members of the first and second binding pairs do not substantially cross-react with any second member of any different binding pair.

The term "binding pair" has been defined previously in the context of the first method of the invention.

In the second method of the invention both primers are labelled with a first member of a binding pair, but the first member of the first binding pair cannot be the same as the first member of the second binding pair; and the second member of the first binding pair cannot be the same as the second member of the second binding pair. Additionally, the first members of the first and second binding pairs do not substantially cross-react with a second member of a different binding pair, i.e. the first member of the first binding pair cannot be substantially bound to the second member of the second binding pair and vice versa. The expression "do not substantially cross-react", as used herein, refers to an amount of binding or recognizing between molecules in an assay mixture under particular assay conditions wherein the second member of the first binding pair is capable of binding or recognizing the first member of the first binding pair and is substantially incapable of binding or recognizing the first member of the second binding pair. Substantial or lack of substantial cross-reaction can be tested using a number of widely known methods, e.g. an enzyme-linked immunosorbent assay (ELISA) or a Western blot assay, or a radioimmunoassay (RIA) or an immunohistochemical assay.

In a preferred embodiment the first member of the first binding pair is an antigen or hapten. Preferably, said hapten is fluorescein isothiocyanate (FITC). In a preferred embodiment the second member of the first binding pair is an antibody specific for the antigen or hapten of the first member of the first binding pair.

In a preferred embodiment the first member of the second binding pair is biotin and the second member of the second binding pair is a biotin-binding molecule.

The expression "biotin-binding molecule", as used herein, refers to any molecule capable of binding to biotin. Preferred biotin-binding molecules include streptavidin and avidin, as well as derivatives and analogues thereof (e.g. nitro-streptavidin) and also antibodies recognizing biotin. In a more preferred embodiment, the biotin-binding molecule is streptavidin.

In an embodiment, the second member of the second binding pair conjugated to magnetic beads is added after step a).

In another embodiment, the second primer labelled at the 5' end with a first member of a second binding pair is bound to the second member of the second binding pair conjugated to magnetic beads before step a) takes place, and wherein said magnetic beads have a size lower than 2.8 µm and are separated from the region of the primer that recognizes the target DNA sequence by a polynucleotide spacer.

In an embodiment, the method for detecting a target DNA sequence according to the second method of the invention involves capturing the amplification products obtained in step a) in the presence of a magnetic bead conjugated to a second member of a second binding pair before step b) takes place.

The expression "amplification product", in the context of the second method of the invention, refers to a double strand amplicon wherein the first strand is labelled at the 5' end with a first member of a first binding pair and the second strand is labelled at the 5' end with a first member of a second binding pair.

The expression "in the presence of a magnetic bead", as used herein, means that a magnetic bead is associated to the second strand of the amplified product either because the second member of the second binding pair conjugated to magnetic beads is added after step a) or because the second primer labelled at the 5' end with a first member of a second binding pair is bound to the second member of the second binding pair conjugated to magnetic beads before step a) takes place.

The presence of a magnetic bead associated to the amplification product is necessary to recover said product from the reaction mixture. This capture is performed by magnetic separation of the products associated to the magnetic beads.

When the second primer labelled at the 5' end with a first member of a second binding pair is bound to the second member of the second binding pair conjugated to magnetic beads before step a) takes place, the product obtained in step a) is washed for removing the excess of primers before step b) takes place. Therefore, in an embodiment, the product obtained in step a) is washed for removing the excess of primers before step b) takes place.

The second method of the invention involves an additional step, wherein the product obtained in step a) is captured with a second member of a first binding pair conjugated to a gold nanoparticle.

The product obtained in step a) of the second method of the invention is a double stranded amplicon wherein the first strand is labelled at the 5' end with a first member of a binding pair and the second strand is associated to a magnetic bead. This product is recovered from the mixture obtained in step a) by interaction with a second member of a first binding pair conjugated to a gold nanoparticle.

The term "conjugated", in the context of step b) of the second method of the invention means that the second member of the first binding pair is coupled to the surface of a gold nanoparticle.

In a preferred embodiment, the product obtained in step a) of the second method of the invention is washed for removing the excess of the second member of a first binding pair conjugated to a gold nanoparticle before step b) takes place.

Step b) of the second method of the invention is the same as step b) of the first method of the invention.

The embodiments of the first method of the invention disclosed above are applicable to the second method of the invention.

Particularly, in an embodiment the target DNA sequence is from a pathogen, preferably from the *Kinetoplastea* class, more preferably *Leishmania* and even more preferably is *L.infantum.* In another embodiment the target DNA sequence is a sequence of the kinetoplast. In another embodiment the sample is from a dog. In another embodiment the recombinase-polymerase isothermal nucleic acid amplification is carried out at a temperature of about 37°C.

### THIRD METHOD OF THE INVENTION

The authors of the present invention have developed a method for detecting a target DNA sequence comprising a RPA and detection by a lateral flow assay.

In a third aspect, the invention relates to an *in vitro* method (hereinafter referred to as "third method of the invention") for detecting a target DNA sequence in a sample comprising the following steps:
a) submitting the sample DNA to a recombinase-polymerase isothermal nucleic acid amplification in the presence of
   (i) a first pair of primers capable of specifically amplifying the target DNA sequence wherein the first primer of the first pair of primers is labelled at the 5' end with a first member of a first binding pair and the second primer of the first pair or primers is labelled at the 5' end with a first member of a second binding pair and
   (ii) a second pair of primers capable of amplifying an endogenous amplification control DNA sequence present in the sample wherein the first primer of the second pair of primers is labelled at the 5' end with said first member of said first binding pair and the second primer of the second pair of primers is labelled at the 5' end with a first member of a third binding pair
      wherein said first members of the first, second and third binding pairs do not substantially cross-react with any second member of any different binding pair and
b) performing a lateral flow assay for the detection of the amplified DNA obtained in step a), wherein
   the amplified DNA obtained in step a) is added to the sample pad of a lateral flow device and
   a second member of the first binding pair associated to a marker capable of generating a colorimetric signal is immobilized on the conjugate pad of a lateral flow device
   and wherein
   the lateral flow device comprises a first area wherein a second member of the second binding pair has been immobilized, a second area wherein a second member of the third binding pair has been immobilized and a third area wherein a reagent capable of specifically binding to the second member of the first binding pair has been immobilized
   wherein the order of the first area and the second area in the lateral flow device is interchangeable
wherein the detection of a colorimetric signal in the first, second and third area of the lateral flow device indicates the presence of the target DNA sequence in the sample, or wherein the absence of a colorimetric signal in the first area and the detection of a colorimetric signal in the second and third area of the lateral flow device indicates the absence of the target DNA sequence in the sample.

The first step of the third method of the invention for detecting a target DNA involves submitting the sample DNA to a multiplexed RPA wherein two further primers are added that allow the detection of an internal control in the same tube. The RPA of the third method of the invention is performed in the presence of a first pair of primers capable of specifically amplifying the target DNA sequence and a second pair of primers capable of specifically amplifying an endogenous amplification control DNA sequence present in the sample.

The expression "endogenous amplification control DNA sequence", as used herein, refers to any DNA sequence present in the sample that can be used to validate the amplification step with different primers than that used for amplifying the target DNA sequence. The endogenous amplification control DNA sequence must be a DNA sequence from the same organism from which the sample is taken. For example, if the sample is a sample from a dog and the target DNA sequence is a sequence from *Leishmania,* the endogenous amplification control DNA sequence must be a sequence from dog DNA.

In a preferred embodiment the endogenous amplification control DNA sequence is a sequence of the 18S rRNA gene. 18S rRNA gene is the gene corresponding to the 18S ribosomal RNA, that is a component of the small eukaryotic ribosomal subunit 40S. 18S rRNA gene sequences from different species can be found in GenBank. Exemplary DNA sequences of 18S rRNA genes are, without limitation, the 18S rRNA gene sequence of cat having GenBank accession numbers AM502841.1 (dated 23 January 2013) or AY150542.1 (dated 1 October 2003); the 18S rRNA gene sequence of dog having GenBank accession numbers FJ797658.1 (dated 10 March 2010), NW_003729148.1 (dated 24 September 2013), AY623831.1 (dated 20 September 2010) or DQ287955.1 (dated 25 November 2006); the 18S rRNA gene sequence of horse having GenBank accession number NR_046271.1 (16 July 2013); the 18S rRNA gene sequence of pig having GenBank accession numbers NR_046261.1 (dated 22 October 2013) or AY265350.1 (dated 1 May 2004); the 18S rRNA gene sequence of human having GenBank accession numbers K03432.1 (dated 3 August 1993), NR_003286.2 (dated 31 January 2014) or X03205.1 (dated 16 December 1994); the 18S rRNA gene sequence of cattle having GenBank accession number NR_036642.1 (dated 8 August 2013); the 18S rRNA gene sequence of sheep having GenBank accession number AY753190.1 (dated 1 October 2006). In a preferred embodiment the 18S rRNA gene is from dog.

The first pair of primers of the third method of the invention is formed by a first primer labelled at the 5' end with a first member of a first binding pair and a second primer labelled at the 5' end with a first member of a second binding pair.

The second pair of primers of the third method of the invention is formed by a first primer labelled at the 5' end with the same first member of the same first binding pair used in the first pair of primers and the second primer is labelled at the 5' end with a first member of a third binding pair.

The first members of the first, second and third binding pairs do not substantially cross-react with a second member of a different binding pair.

In an embodiment, the first member of the first binding pair is an antigen or a hapten. In a preferred embodiment the hapten is fluorescein isothiocyanate (FITC). In an embodiment, the second member of the first binding pair is an antibody specific for said antigen, preferably a rabbit or goat antibody, more preferably a goat anti-FITC antibody.

In an embodiment, the first member of the second binding pair is biotin. In an embodiment, the second member of the second binding pair is a biotin-binding molecule, preferably streptavidin.

In an embodiment the first member of the third binding pair is an antigen or a hapten. In a preferred embodiment the hapten is digoxigenin. In an embodiment the second member of the third binding pair is an antibody specific for said antigen.

The second step of the third method of the invention involves performing a lateral flow assay for the detection of the amplified DNA obtained in step a).

The expression "lateral flow assay", as used herein, refers to an assay or test intended to detect and/or quantify the presence or absence of a target DNA sequence in a sample based on a lateral flow device. A lateral flow assay is particularly useful for point of care testing since it does not require specialized and costly equipment.

The expression "lateral flow device", as used herein, refers to a device comprising a series of capillary beds that have the capacity to transport fluids spontaneously. This device is designed to permit a continuous, lateral movement of a particular test sample fluid from the proximal end of the lateral flow device to the distal end of the lateral flow device. The elements of a lateral flow device according to the invention are a sample pad, a conjugate pad, two different test lines or test zones and a control line or control zone. The two test lines are located after the conjugate pad, and the control line is located after the two test lines. In general, a marker capable of generating a signal is solubilized and bound to an antigen or antibody in the sample, subsequent to which it moves through the lateral flow device via capillary action. It may then bind to the amplicons, as present, after which it further binds to a second immobilized antibody or antigen, along a test line or the like, at which point a signal forms which may be read by eye or machine.

The lateral flow device includes conventional dipsticks which may be used vertically, as well as devices in which membranes are fixed in a horizontal position so that flow along the membrane occurs horizontally or laterally. The lateral flow device may be made of bibulous materials such as a porous paper, a sintered polymer, a glass fibre membrane, nitrocellulose or cellulose. A lateral flow device made of microstructured plastic is also encompassed by the present invention. The lateral flow device may have different geometries. In a preferred embodiment the lateral flow device is a test strip, more preferably a nitrocellulose membrane.

The lateral flow device according to the present invention may be provided with a backing. The backing may be a rigid or semi-rigid polymer, e.g. polyester, vinyl, etc, to provide mechanical strength.

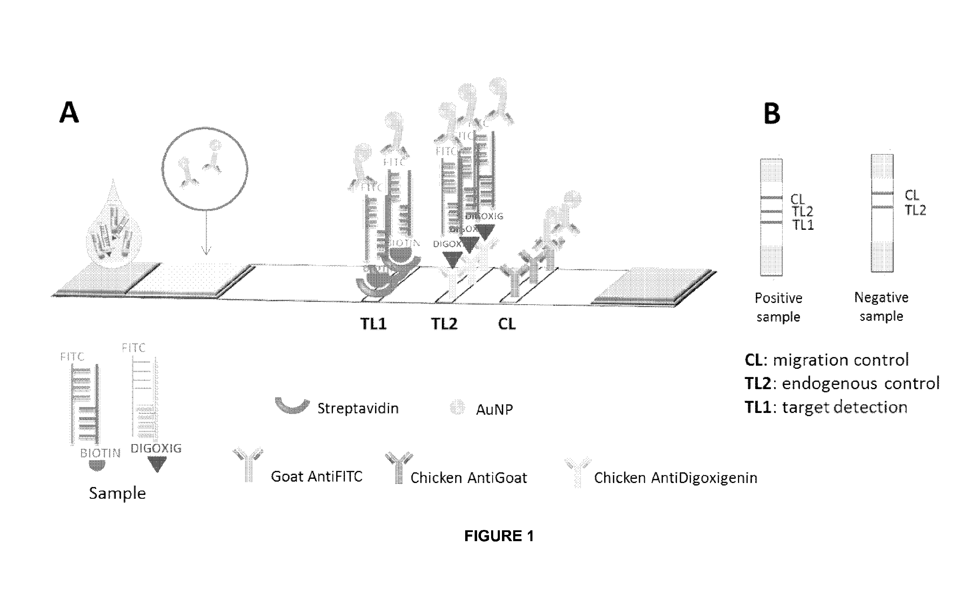

The examples of the present invention show a dipstick designed for simultaneous detection of two different amplicons: one labelled with FITC and biotin, corresponding to the amplicon of the target DNA sequence, and the second one labelled with FITC and digoxigenin, corresponding to an endogenous control. The double labelled amplicons bound first to gold-labelled FITC-specific antibodies in the conjugate pad of the dipstick. The gold complexes diffused over the membrane by capillarity. Only the amplicons captured by gold particles bound when they overflow the immobilized biotin-ligand or anti-digoxigenin antibody molecules at the respective test band and generated there a red-blue band over the time. Not captured gold particles flowed over the control band and were fixed there by species-specific antibodies. A schematic diagram of this method is shown in Figure 1.

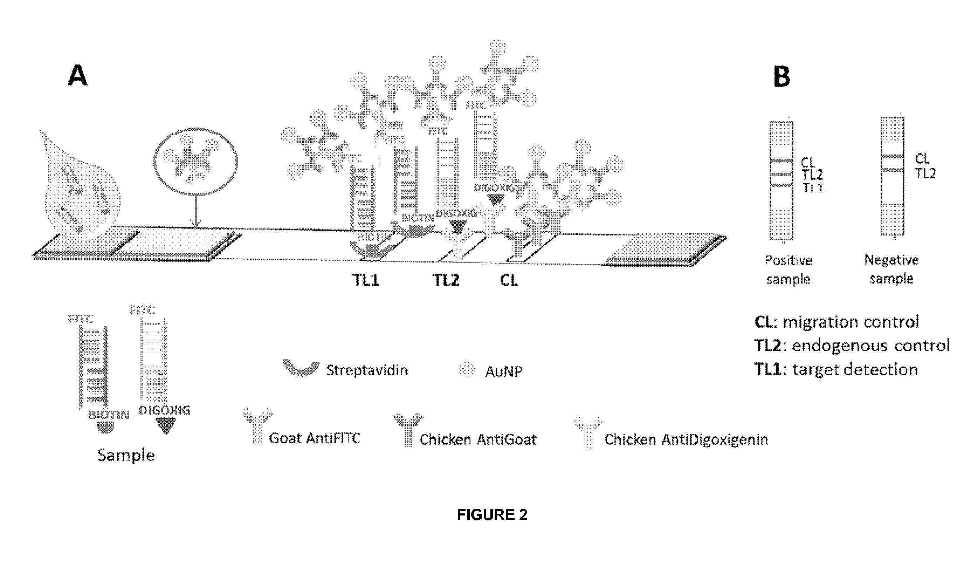

The examples of the present invention also show an enhanced lateral flow assay based on the immobilization on the conjugate pad of primary antibodies specific to the amplicons and secondary antibodies labelled with AuNPs which recognize the primary ones. In this lateral flow approach, secondary antibodies (chicken anti-goat IgG) were labelled with AuNPs which can recognize a primary one that acts as detection antibody (goat anti-FITC) forming a complex which was dispensed onto the conjugate pad. The test line of the nitrocellulose membrane contained a streptavidin solution and the control line contained an anti-species solution. When the sample started to flow through the strip, the complex of double antibody was released, producing the capture of the isothermally amplified DNA through the strand labeled with FITC. While the sample continued migrating through the membrane, the test line became visible due to the binding of streptavidin with the biotin present in the isothermally amplified DNA. The endogenous control was also visualized in the second test line due to the recognition of the digoxigenin present in the amplified DNA by the specific antibodies immobilized in the strip. The control line turned visible when the anti-species antibodies captured any excess of antibody labelled with gold nanoparticles. A schematic diagram of this method is shown in Figure 2.

In the lateral flow assay of the present invention, the amplified DNA obtained in step a) of the third method of the invention is added to the sample pad of a lateral flow device.

The term "amplified DNA", in the context of the third method of the invention, refers to two different double stranded amplicons: (i) a double stranded amplicon corresponding to the amplified target DNA wherein the first strand is labelled at the 5' end with a first member of a first binding pair and the second strand is labelled at the 5' end with a first member of a second binding pair; and (ii) a double stranded amplicon corresponding to the amplified endogenous control DNA wherein the first strand is labelled at the 5' end with the same first member of same first binding pair as the first strand of the double stranded amplicon corresponding to the amplified target DNA and the second strand is labelled at the 5' end with a first member of a third binding pair.

The term "sample pad", in the context of the present invention, refers to a region of the lateral flow device wherein the sample mixture containing the amplified product in step a) is deposited and that acts as a sponge holding an excess of sample fluid.

Once soaked, the fluid of the sample mixture migrates to the next element of the lateral flow device.

The lateral flow device of the invention has a second member of the first binding pair associated to a marker capable of generating a colorimetric signal immobilized in the conjugate pad of a lateral flow device.

The expression "marker capable of generating a colorimetric signal", as used herein, refers to a molecule that is capable of producing color or photoluminescence which can be used for detecting a signal in the first, second and/or third area of the lateral flow device. Examples of markers capable of generating a colorimetric signal are, without limitation, gold nanoparticles, quantum dots, carbon nanotubes, magnetic nanoparticles, liposomes, enzymes, coloured particles such as coloured latex microparticles, colloidal carbon particles and metallic colloids such as gold and silver. This definition also includes fluorophores such as cyanine, fluorescein, rhodamine, Alexa fluors, Dylight fluors, ATTO dyes, BODIPY dyes, SETA dyes and SeTau dyes, that may be attached to another molecule by chemically reactive derivatives of said fluorophores (e.g. FITC, TRITC, NHS-fluorescein, fluorescein-5-maleimide, 6-FAM-phosphoramidite, etc.). In a preferred embodiment the marker capable of generating a colorimetric signal is a gold nanoparticle.

In an embodiment the marker capable of generating a colorimetric signal is conjugated to the second member of the first binding pair. The term "conjugated", as used herein, means that the second member of the first binding pair is coupled to the marker capable of generating a colorimetric signal.

The authors of the present invention have developed an enhanced lateral flow assay in which primary antibodies specific to the amplicons and secondary antibodies labelled with a marker capable of generating a colorimetric signal which recognize the primary ones are immobilized on the conjugation pad.

Therefore, in another embodiment the marker capable of generating a colorimetric signal is conjugated to an antibody which binds specifically to the second member of the first binding pair.

The term "immobilized", as used herein, means that the second member of the first binding pair is attached to the surface of dried bio-active particles that are embedded in a salt-sugar matrix on the conjugate pad of the lateral flow device. The salt-sugar matrix contains everything to guarantee an optimized chemical reaction between the first member of the first binding pair of the amplicons obtained in step a) and its chemical partner. While the sample fluid dissolves the salt-sugar matrix, it also dissolves the particles and the amplicons bind to the particles while migrating further through the capillary bed. The second member of the first binding pair binds to the first strand of both amplicons (the amplicon corresponding to the amplified target DNA and the amplicon corresponding to the endogenous control).

The term "associated", as used herein, means that the second member of the first binding pair can be directly coupled to a marker capable of generating a colorimetric signal forming a conjugate or that the second member of the first binding pair is bound to another molecule that is directly coupled to a marker capable of generating a colorimetric signal.

The second member of the first binding pair associated to a marker capable of generating a colorimetric signal is immobilized in the conjugate pad.

The term "conjugate pad", as used herein, refers to a region of the lateral flow device situated between the sample pad and before the test lines and the control line wherein the second member of the first binding pair is attached to the surface of dried bio-active particles that are embedded in a salt-sugar matrix. The salt-sugar matrix contains everything to guarantee an optimized chemical reaction between the first member of the first binding pair of the amplicons obtained in step a) and its chemical partner. While the sample fluid dissolves the salt-sugar matrix, it also dissolves the particles and the amplicons bind to the particles while migrating further through the capillary bed. The second member of the first binding pair binds to the first strand of both amplicons (the amplicon corresponding to the amplified target DNA and the amplicon corresponding to the endogenous control).

The lateral flow device of the present invention comprises a first, a second and a third area. The first and second areas are located after the conjugate pad and before the third area, but the order of the first area and the second area in the lateral flow device is interchangeable. This means that, after the sample fluid dissolves the particles on the conjugate pad, the fluid can migrate to the next element in the lateral flow device and said next element may be the first area or the second area. The order between said areas may be first area-second area-third area or second area-first area-third area. In a preferred embodiment, the order is first area-second area-third area. In another embodiment, the order is second area-first area-third area.

The lateral flow device of the present invention comprises a first area wherein a second member of the second binding pair has been immobilized.

The term "first area", as used herein, refers to a first test line or test zone wherein a second member of the second binding pair has been immobilized and that only captures those particles onto which the amplicon corresponding to the amplified target DNA has been immobilized. By the time the sample-conjugate mix reaches this area, the amplicon has been bound to the particle and the second member of the second binding pair binds the complex. After a while, when more and more fluid has passed this area, particles accumulate and this area changes color. This is the area wherein the colorimetric signal for the identification and/or quantification of the target DNA is obtained.

The second member of the second binding pair is immobilized in the first area of the lateral flow device. However, said immobilization is a covalent binding different from the immobilization on the conjugate pad. Therefore, once the amplicon corresponding to the amplified target DNA is bound to the second member of the second binding pair, the complex formed remains in the first area and does not flow until the next element of the lateral flow device. The compounds of the sample not bound to the first area migrate to the next element of the lateral flow device.

The lateral flow device of the present invention comprises a second area wherein a second member of the third binding pair has been immobilized.

The term "second area", as used herein, refers to a second test line or test zone wherein a second member of the third binding pair has been immobilized and that only captures those particles onto which the amplicon corresponding to the amplified endogenous control DNA has been immobilized. By the time the sample-conjugate mix reaches this area, the amplicon has been bound to the particles and the second member of the third binding pair binds the complex. After a while, when more and more fluid has passed this area, particles accumulate and this area changes color. This is the area wherein the colorimetric signal for the identification and/or quantification of the endogenous control DNA is obtained.

The second member of the third binding pair is immobilized in the second area of the lateral flow device. However, said immobilization is a covalent binding different from the immobilization on the conjugate pad. Therefore, once the amplicon corresponding to the endogenous control DNA is bound to the second member of the third binding pair, the complex formed remains in the second area and does not flow until the next element of the lateral flow device. The compounds of the sample not bound to the second area migrate to the next element of the lateral flow device.

The lateral flow device of the present invention comprises a third area wherein a reagent capable of specifically binding to the second member of the first binding pair has been immobilized.

The term "third area", as used herein, refers to a control line or control zone wherein a reagent capable of specifically binding to the second member of the first binding pair has been immobilized and that only captures those particles onto which none of the amplicons have been immobilized. The reagent binds those particles and after a while, when more and more fluid has passed this area, particles accumulate and this area changes color. This is the area wherein the colorimetric signal for the identification and/or quantification of the migration control is obtained and that shows that migration conditions and lateral flow technology worked fine.

The reagent capable of specifically binding to the second member of the first binding pair is immobilized in the third area of the lateral flow device. However, said immobilization is a covalent binding different from the immobilization on the conjugate pad. Therefore, once the particles not bound to an amplicon are bound to said reagent, the complex formed remains in the third area and does not flow until the next element of the lateral flow device. The compounds of the sample not bound to the second area migrate to the distal end of the lateral flow device entering the final porous material that simply acts as a waste container.

The lateral flow device may contain additional areas wherein additional target DNA molecules and/or controls may be detected.

The term "reagent capable of specifically binding to the second member of the first binding pair", as used herein, refers to a molecule that is capable of binding to the second member of the first binding pair but it is not capable of binding to other members different from the second member of the first binding pair, thus allowing a specific detection of the molecules bound to the second member of the first binding pair. In a preferred embodiment, the reagent capable of binding to the second member of the first binding pair is a species-specific antibody.

The expression "species-specific antibody", as used herein, refers to a secondary antibody that recognizes only one host species of primary antibody. Secondary antibodies are generated by immunizing a host animal with an antibody from a different species. For example, immunizing a chicken with purified goat IgG will generate chicken anti-goat IgG antibodies that will bind to all classes of goat IgG, as well as any other molecules sharing the same conserved domains (e.g. IgM). Goat, donkey, sheep, chicken and rabbit are the most commonly used host species for raising secondary antibodies, though others are available. In a preferred embodiment, the species-specific antibody is anti-goat IgG, preferably chicken anti-goat IgG.

The first, second and third area of the lateral flow device of the present invention may be a line or a region having any shape, such as an area having different geometrical shapes (a circular area, a triangular area, a rectangular area, a hexagonal area, etc.) or an area having a non-geometrical shape (i.e. a shape with irregular contours). In a preferred embodiment, the first, second and third areas of the lateral flow device are a first, second and third lines.

In the third method of the invention the detection of a colorimetric signal in the first, second and third area of the lateral flow device indicates the presence of the target DNA sequence and the absence of a colorimetric signal in the first area and the detection of a colorimetric signal in the second and third area of the lateral flow device indicates the absence of the target DNA sequence in the sample. The absence of a colorimetric signal in the second area indicates that the DNA extraction or the amplification step has not worked. The absence of a colorimetric signal in the third area indicates that the migration on the lateral flow assay has not worked.

Depending on the marker used the colorimetric signal may be visible to the naked eye or it may be detected by a fluorescence reader.

The terms "target DNA sequence", "sample", "recombinase-polymerase isothermal nucleic acid amplification", "primer", "capable of specifically amplifying", "5' end", "binding pair", "antigen", "hapten", "specific" and "gold nanoparticle " have been defined previously in the context of the first method of the invention.

The terms "do not substantially cross-react" and "biotin-binding molecule" have been defined previously in the context of the second method of the invention.

The embodiments of the first and second method of the invention disclosed above are applicable to the third method of the invention.

Particularly, in an embodiment the target DNA sequence is from a pathogen, preferably from the *Kinetoplastea* class, more preferably *Leishmania,* even more preferably *L.infantum.* In another embodiment the target DNA sequence is a sequence of the kinetoplast. In another embodiment the sample is from a dog. In another embodiment the recombinase-polymerase isothermal nucleic acid amplification is carried out at a temperature of about 37°C.

### KITS OF THE INVENTION

The invention also includes kits for carrying out the methods according to the invention.

The term "kit", as used in the present document, refers to a combination of a set of reagents suitable for detecting a target DNA by a method according to the invention together with one or more types of elements or components (for example, other types of biochemical reagents, containers, packaging suitable for its commercial sale, substrates to which the reagents are bound, electronic hardware components, etc.).

The invention relates to kits for carrying out the first method of the invention wherein one of the primers is labelled with a gold nanoparticle and the other primer is associated to magnetic beads.

In an aspect, the invention relates to a kit (hereinafter referred to as "first kit of the invention") comprising a pair of primers capable of specifically amplifying a target DNA sequence wherein the first primer is labelled at the 5' end with a gold nanoparticle and the second primer is labelled at the 5' end with a first member of a binding pair, and the first member of a binding pair is bound to a second member of a binding pair conjugated to magnetic beads, and wherein said magnetic beads have a size lower than 2.8 µm and are separated from the region of the primer that recognizes the target DNA sequence by a polynucleotide spacer.

The invention also relates to kits for carrying out the first method of the invention wherein the magnetic beads conjugated to a second member of a binding pair are provided separated from the primer originally used in the amplification step.

In an aspect, the invention relates to a kit (hereinafter referred to as "second kit of the invention") comprising
(i) a pair of primers capable of specifically amplifying a target DNA sequence wherein the first primer is labelled at the 5' end with a gold nanoparticle and the second primer is labelled at the 5' end with a first member of a binding pair, and
(ii) a second member of a binding pair conjugated to magnetic beads.

In an embodiment of the first and second kits of the invention the first member of a binding pair is an antigen o hapten, preferably biotin. In another embodiment of the first and second kits of the invention the second member of the binding pair is an antibody specific for said antigen. In another embodiment of the first and second kits of the invention the second member of a binding pair is streptavidin.

In an embodiment of the first and second kits of the invention the magnetic beads are superparamagnetic microbeads.

In an embodiment of the first and second kits of the invention the kit further comprises at least a reagent selected from a recombinase, a single-stranded DNA binding protein and a strand-displacing DNA polymerase.

The term "recombinase", as used herein, refers to an enzyme that can coat single-stranded DNA (ssDNA) to form filaments, which can then scan double-stranded DNA (dsDNA) for regions of sequence homology. When homologous sequences are located, the nucleoprotein filament (comprising the recombinase) strand invades the dsDNA creating a short hybrid and a displaced strand bubble known as a D-loop. Suitable recombinase agents include the *E.coli* RecA protein, the T4 uvsX protein, or any homologous protein or protein complex from any phyla. Eukaryotic RecA homologues are generally named Rad51 after the first member of this group to be identified. Other non-homologous recombinases may be utilized in place of RecA, for example as RecT or RecO. Recombinase agents generally require the presence of ATP, ATPγS, or other nucleoside triphosphates and their analogs. Naturally, any derivatives and functional analogs of the recombinase above may also function itself as a recombinase and these derivatives and analogs are also contemplated in the present invention. For example, a small peptide from RecA, which has been shown to retain some aspects of the recombination properties of RecA, may be used.

The term "single-stranded DNA binding protein", as used herein, refers to a protein that binds to single-stranded DNA, melt secondary structure, facilitate outgoing strand displacement, and suppress branch migration. Examples of single-stranded DNA binding proteins useful for the invention are, without limitation, *E.coli* SSB and bacteriophages T4 gp32.

The term "strand-displacing DNA polymerase", as used herein, refers to a DNA polymerase having the ability to displace downstream DNA encountered during synthesis. Examples of strand-displacing DNA polymerases are, without limitation, *E.coli* polymerase II or III, *E.coli* polymerase V and □29 polymerase.

In another embodiment of the first and second kits of the invention the kit further comprises a screen-printed carbon electrode.

The invention also relates to kits for carrying out the third method of the invention including a pair of primers for the amplification of the target DNA sequence, a pair of primers for the amplification of the endogenous amplification control DNA sequence and reagents for performing the RPA.

Therefore, in an aspect the invention relates to a kit (hereinafter referred to as "third kit of the invention") comprising:
(i) a first pair of primers capable of specifically amplifying a target DNA sequence wherein the first primer of the first pair of primers is labelled at the 5' end with a first member of a first binding pair and the second primer of the first pair of primers is labelled at the 5' end with a first member of a second biding pair,
(ii) a second pair of primers capable of amplifying an endogenous amplification control DNA sequence wherein the first primer of the second pair of primers is labelled at the 5' end with said first member of said first binding pair and the second primer of the second pair of primers is labelled at the 5' end with a first member of a third binding pair,
   wherein said first members of the first, second and third binding pairs do not substantially cross-react with any second member of any different binding pair,
(iii) a recombinase,
(iv) a single-stranded DNA binding protein and
(v) a strand-displacing DNA polymerase.

In an embodiment of the third kit of the invention the first member of the first binding pair is an antigen or a hapten, preferably the hapten is fluorescein isothiocyanate (FITC). In another embodiment of the third kit of the invention the second member of the first binding pair is an antibody specific for said antigen, preferably a rabbit or goat antibody.

In an embodiment of the third kit of the invention the endogenous amplification control DNA sequence is from the same organism than the organism from which the target DNA sequence to be detected derives. Preferably, the endogenous amplification control DNA sequence is a sequence of the 18S rRNA gene, more preferably the 18S rRNA gene is from dog.

If the endogenous amplification control DNA sequence is a sequence of the dog 18S rRNA gene, an embodiment of the third kit of the invention is a kit wherein the pair of primers capable of amplifying the endogenous amplification control DNA sequence are primers of sequence SEQ ID NO: 3 and SEQ ID NO: 4.

In an embodiment, the third kit of the invention further comprises a second member of the first binding pair conjugated to a marker capable of generating a colorimetric signal, preferably a gold nanoparticle.

In another embodiment, the third kit of the invention further comprises a second member of the first binding pair and an antibody which binds specifically to the second member of the first binding pair, wherein said antibody is conjugated to a marker capable of generating a colorimetric signal, preferably a gold nanoparticle.

In another embodiment, the third kit of the invention further comprises a lateral flow device for simultaneous detection of two amplification DNA products having:
(i) a second member of the second binding pair immobilized in a first area of the lateral flow device;
(ii) a second member of the third binding pair immobilized in a second area of the lateral flow device; and
(iii) a reagent capable of specifically binding to the second member of the first binding pair immobilized in a third area of the lateral flow device
   wherein the order of the first area and the second area in the lateral flow device is interchangeable.

In a preferred embodiment of the third kit of the invention the first member of the second binding pair is biotin and the second member of the second binding pair is a biotin-binding molecule, preferably streptavidin.

In a preferred embodiment of the third kit of the invention the first member of the third binding pair is an antigen or a hapten, preferably digoxigenin. In a preferred embodiment of the third kit of the invention the second member of the third binding pair is an antibody specific for said antigen.

In an embodiment of the third kit of the invention the reagent capable of specifically binding to the second member of the first binding pair is a species-specific antibody.

In an embodiment of the third kit of the invention the lateral flow device is a test strip, preferably a nitrocellulose membrane.

If the target DNA sequence is a sequence from the kinetoplast of *L.infantum,* an embodiment of the first, second and third kits of the invention is a kit wherein the pair of primers capable of specifically amplifying the target DNA sequence are primers of sequence SEQ ID NO: 1 and SEQ ID NO: 2.

The terms disclosed in this section have been defined in the context of the first, second and third methods of the invention.

The embodiments of the first, second and third methods of the invention disclosed above are applicable to the kits of the invention.

Particularly, in an embodiment the target DNA sequence is from a pathogen, preferably from the *Kinetoplastea* class, more preferably *Leishmania,* even more preferably *L.infantum.* In another embodiment the target DNA sequence is a sequence of the kinetoplast.

### OLIGONUCLEOTIDES OF THE INVENTION

The invention also discloses oligonucleotides to be used in the methods of the invention and to be included in kits.

In an aspect the invention relates to an oligonucleotide whose sequence is selected from the group consisting of SEQ ID NO: 1, SEQ ID NO: 2, SEQ ID NO: 3 and SEQ ID NO: 4.

In a preferred embodiment said oligonucleotide is labelled at the 5' end with a gold nanoparticle or with a first member of a binding pair.

### DIAGNOSTIC METHODS OF THE INVENTION

The methods of the present invention for detecting a target DNA sequence in a sample allow diagnosing an infection by a pathogen in a subject.

In an aspect, the invention relates to an *in vitro* method for diagnosing an infection by a pathogen in a subject comprising detecting the presence of a target DNA sequence from said pathogen in a biological sample of said subject by a method according to the invention.

In the context of the present invention, *"in vitro* method for diagnosing an infection" is understood as a method which allows showing the existence of an infection in a subject by means of detecting the presence of a target DNA sequence from an infectious pathogen in a sample isolated from the subject.

The term "infection" or "infectious disease", as used herein, refers to the invasion of a host organism by an infectious agent such as viruses, viroids and prions, microorganisms such as bacteria, nematodes such as roundworms and pinworms, arthropods, fungi and parasites.

The term "pathogen", as used herein, refers to an infectious agent such as viruses, viroids and prions, microorganisms such as bacteria, nematodes such as roundworms and pinworms, arthropods, fungi and parasites. Exemplary pathogens are disclosed in the context of the first method of the invention.

"Subject" in the present invention is understood as any animal, preferably an animal classified as mammal and includes but is not limited to domestic and farm animals, primates and humans, for example human beings, non-human primates, cows, horses, pigs, sheep, goats, dogs, cats or rodents. Preferably, the subject is a female or male human being of any race or age. Most preferably, the subject is a dog. In the context of the present invention, the subject is a subject who potentially suffers from an infection caused by a pathogen.

The term "biological sample", as used herein, refers to animal or human samples including, without limitation, any biological fluid (blood, bone marrow, plasma, serum, bronchoalveolar washing fluid, urine, nasal secretion, ear secretion, urethral secretion, cerebrospinal fluid, pleural fluid, synovial fluid, peritoneal fluid, sputum, lymph, plasma, ejaculate, lung aspirate, etc.), cell, tissue, organ or portion thereof that contains DNA. Preferably, the biological sample is selected from lesional swab, throat swab, nasal swab, vaginal swab and rectal swab. A biological sample can include a neoplastic cell. Said samples can be obtained by conventional methods, using processes known in the state of the art by the person skilled in the art.

All the embodiments of the first, second and third methods of the invention disclosed above are applicable to the diagnostic methods of the invention.

The present invention is also directed to:
[1]. An *in vitro* method for detecting a target DNA sequence in a sample comprising the following steps:
   a) submitting the sample DNA to a recombinase-polymerase isothermal nucleic acid amplification in the presence of a pair of primers capable of specifically amplifying the target DNA sequence wherein the first primer is labelled at the 5' end with a gold nanoparticle and the second primer is labelled at the 5' end with a first member of a binding pair, and
   b) measuring the electrochemical signal produced by the gold nanoparticles forming part of the amplification products obtained in step a) using a screen-printed carbon electrode after capture of the amplification products on the electrode using magnetic beads conjugated to a second member of a binding pair,
   wherein the detection of an electrochemical signal superior to the background signal indicates the presence of the target DNA sequence in the sample.
[2]. The method according to [1], wherein the first member of a binding pair is an antigen or hapten and the second member of the binding pair is an antibody specific for said antigen.
[3]. The method according to [1] or [2], wherein the second primer labelled at the 5' end with a first member of a binding pair is bound to the second member of the binding pair conjugated to magnetic beads before step a) takes place, and wherein said magnetic beads have a size lower than 2.8 µm and are separated from the region of the primer that recognizes the target DNA sequence by a polynucleotide spacer.
[4]. The method according to [1] or [2], wherein the second member of the binding pair conjugated to magnetic beads is added after step a).
[5]. The method according to [1], [2] or [3], wherein the product obtained in step a) is washed for removing the excess of primers before step b) takes place.
[6]. The method according to any of [1] to [5], wherein the first member of a binding pair is biotin and the second member of a binding pair is streptavidin.
[7]. An *in vitro* method for detecting a target DNA sequence in a sample comprising the following steps:
   a) submitting the sample DNA to a recombinase-polymerase isothermal nucleic acid amplification in the presence of a pair of primers capable of specifically amplifying the target DNA sequence wherein the first primer is labelled at the 5' end with a first member of a first binding pair and the second primer is labelled at the 5' end with a first member of a second binding pair, and
      wherein said first members of the first and second binding pairs do not substantially cross-react with any second member of any different binding pair, and
   b) measuring the electrochemical signal produced by the gold nanoparticles forming part of the amplification products obtained in step a) using a screen-printed carbon electrode after the first member of a first binding pair is bound to a second member of a first binding pair conjugated to a gold nanoparticle and after capture of the amplification products on the electrode using magnetic beads conjugated to a second member of a second binding pair
      wherein the detection of an electrochemical signal superior to the background signal indicates the presence of the target DNA sequence in the sample.
[8]. The method according to [7], wherein the product obtained after capturing the amplified product with a second member of a first binding pair conjugated to a gold nanoparticle is washed before step b) takes place.
[9]. The method according to [7] or [8], wherein the first member of the first binding pair is an antigen or hapten and the second member of the first binding pair is an antibody specific for said antigen.
[10]. The method according to [9], wherein the hapten is fluorescein isothiocyanate (FITC).
[11]. The method according to any of [7] to [10], wherein the first member of the second binding pair is biotin and the second member of the second binding pair is a biotin-binding molecule.
[12]. The method according to [11], wherein the biotin-binding molecule is streptavidin.
[13]. The method according to any of [7] to [12], wherein the second member of the second binding pair conjugated to magnetic beads is added after step a).
[14]. The method according to any of [7] to [12], wherein the second primer labelled at the 5' end with a first member of a second binding pair is bound to the second member of the second binding pair conjugated to magnetic beads before step a) takes place, and wherein said magnetic beads have a size lower than 2.8 µm and are separated from the region of the primer that recognizes the target DNA sequence by a polynucleotide spacer.
[15]. An *in vitro* method for detecting a target DNA sequence in a sample comprising the following steps:
   a) submitting the sample DNA to a recombinase-polymerase isothermal nucleic acid amplification in the presence of
      (i) a first pair of primers capable of specifically amplifying the target DNA sequence wherein the first primer of the first pair of primers is labelled at the 5' end with a first member of a first binding pair and the second primer of the first pair or primers is labelled at the 5' end with a first member of a second binding pair and
      (ii) a second pair of primers capable of amplifying an endogenous amplification control DNA sequence present in the sample wherein the first primer of the second pair of primers is labelled at the 5' end with said first member of said first binding pair and the second primer of the second pair of primers is labelled at the 5' end with a first member of a third binding pair
         wherein said first members of the first, second and third binding pairs do not substantially cross-react with any second member of any different binding pair and
   b) performing a lateral flow assay for the detection of the amplified DNA obtained in step a), wherein the amplified DNA obtained in step a) is added to the sample pad of a lateral flow device and
      a second member of the first binding pair associated to a marker capable of generating a colorimetric signal is immobilized on the conjugate pad of a lateral flow device
      and wherein
      the lateral flow device comprises a first area wherein a second member of the second binding pair has been immobilized, a second area wherein a second member of the third binding pair has been immobilized and a third area wherein a reagent capable of specifically binding to the second member of the first binding pair has been immobilized
      wherein the order of the first area and the second area in the lateral flow device is interchangeable
   wherein the detection of a colorimetric signal in the first, second and third area of the lateral flow device indicates the presence of the target DNA sequence in the sample, or wherein the absence of a colorimetric signal in the first area and the detection of a colorimetric signal in the second and third area of the lateral flow device indicates the absence of the target DNA sequence in the sample.
[16]. The method according to [15], wherein the first member of the first binding pair is an antigen or a hapten and the second member of the first binding pair is an antibody specific for said antigen.
[17]. The method according to [16], wherein the antibody is a rabbit or goat antibody.
[18]. The method according to [16] or [17], wherein the hapten is fluorescein isothiocyanate (FITC).
[19]. The method according to any of [15] to [18], wherein the first member of the second binding pair is biotin and the second member of the second binding pair is a biotin-binding molecule.
[20]. The method according to [19], wherein the biotin-binding molecule is streptavidin.
[21]. The method according to any of [15] to [20], wherein the first member of the third binding pair is an antigen or a hapten and the second member of the third binding pair is an antibody specific for said antigen.
[22]. The method according to [21], wherein the hapten is digoxigenin.
[23]. The method according to any of [15] to [22], wherein the reagent capable of binding to the second member of the first binding pair is a species-specific antibody.
[24]. The method according to any of [15] to [23], wherein the marker capable of generating a colorimetric signal is a gold nanoparticle.
[25]. The method according to any of [15] to [24], wherein the marker capable of generating a colorimetric signal is conjugated to the second member of the first binding pair.
[26]. The method according to any of [15] to [24], wherein the marker capable of generating a colorimetric signal is conjugated to an antibody which binds specifically to the second member of the first binding pair.
[27]. The method according to any of [15] to [26], wherein the lateral flow device is a test strip.
[28]. The method according to [27], wherein the test strip is a nitrocellulose membrane.
[29]. The method according to any of [1] to [28], wherein the target DNA sequence is from a pathogen.
[30]. The method according to [29], wherein the pathogen is an organism from the *Kinetoplastea* class.
[31]. The method according to [30], wherein the organism from the *Kinetoplastea* class is *Leishmania.*
[32]. The method according to [31], wherein the *Leishmania* is *Leishmania infantum.*
[33]. The method according to any of [30] to [32], wherein the target DNA sequence is a sequence of the kinetoplast.
[34]. The method according to any of [15] to [33], wherein the endogenous amplification control DNA sequence is a sequence of the 18S rRNA gene.
[35]. The method according to [34], wherein the 18S rRNA gene is from dog.
[36]. The method according to [1] to [35], wherein the sample is from a dog.
[37]. The method according to any of [1] to [36], wherein the recombinase-polymerase isothermal nucleic acid amplification is carried out at a temperature of about 37°C.
[38]. A kit comprising a pair of primers capable of specifically amplifying a target DNA sequence wherein the first primer is labelled at the 5' end with a gold nanoparticle and the second primer is labelled at the 5' end with a first member of a binding pair, and the first member of a binding pair is bound to a second member of a binding pair conjugated to magnetic beads, and wherein said magnetic beads have a size lower than 2.8 µm and are separated from the region of the primer that recognizes the target DNA sequence by a polynucleotide spacer.
[39]. A kit comprising:
   (i) a pair of primers capable of specifically amplifying a target DNA sequence wherein the first primer is labelled at the 5' end with a gold nanoparticle and the second primer is labelled at the 5' end with a first member of a binding pair, and
   (ii) a second member of a binding pair conjugated to magnetic beads.
[40]. The kit according to [38] or [39], wherein the first member of a binding pair is an antigen or hapten and the second member of the binding pair is an antibody specific for said antigen.
[41]. The kit according to [38] or [39], wherein the first member of a binding pair is biotin and the second member of a binding pair is streptavidin.
[42]. The kit according to any of [38] to [41], wherein the magnetic beads are superparamagnetic microbeads.
[43]. The kit according to any of [38] to [42] further comprising at least a reagent selected from a recombinase, a single-stranded DNA binding protein and a strand-displacing DNA polymerase.
[44]. The kit according to any of [38] to [43] further comprising a screen-printed carbon electrode.
[45]. A kit comprising:
   (i) a first pair of primers capable of specifically amplifying a target DNA sequence wherein the first primer of the first pair of primers is labelled at the 5' end with a first member of a first binding pair and the second primer of the first pair of primers is labelled at the 5' end with a first member of a second biding pair,
   (ii) a second pair of primers capable of amplifying an endogenous amplification control DNA sequence wherein the first primer of the second pair of primers is labelled at the 5' end with said first member of said first binding pair and the second primer of the second pair of primers is labelled at the 5' end with a first member of a third binding pair,
      wherein said first members of the first, second and third binding pairs do not substantially cross-react with any second member of any different binding pair,
   (iii) a recombinase,
   (iv) a single-stranded DNA binding protein and
   (v) a strand-displacing DNA polymerase.
[46]. The kit according to [45] further comprising a second member of the first binding pair conjugated to a marker capable of generating a colorimetric signal.
[47]. The kit according to [45] further comprising a second member of the first binding pair and an antibody which binds specifically to the second member of the first binding pair, wherein said antibody is conjugated to a marker capable of generating a colorimetric signal.
[48]. The kit according to any of [45] to [47], wherein the first member of the first binding pair is an antigen or a hapten and the second member of the first binding pair is an antibody specific for said antigen.
[49]. The kit according to [48], wherein the antibody is a rabbit or goat antibody.
[50]. The kit according to [48] or [49], wherein the hapten is fluorescein isothiocyanate (FITC).
[51]. The kit according to any of [46] to [50], wherein the marker capable of generating a colorimetric signal is a gold nanoparticle.
[52]. The kit according to any of [45] to [51] further comprising a lateral flow device for simultaneous detection of two amplification DNA products having:
   (i) a second member of the second binding pair immobilized in a first area of the lateral flow device;
   (ii) a second member of the third binding pair immobilized in a second area of the lateral flow device; and
   (iii) a reagent capable of specifically binding to the second member of the first binding pair immobilized in a third area of the lateral flow device
      wherein the order of the first area and the second area in the lateral flow device is interchangeable.
[53]. The kit according to [52], wherein the first member of the second binding pair is biotin and the second member of the second binding pair is a biotin-binding molecule.
[54]. The kit according to [53], wherein the biotin-binding molecule is streptavidin.
[55]. The kit according to any of [52] to [54], wherein the first member of the third binding pair is an antigen or a hapten and the second member of the third binding pair is an antibody specific for said antigen.
[56]. The kit according to [55], wherein the hapten is digoxigenin.
[57]. The kit according to any of [52] to [56], wherein the reagent capable of specifically binding to the second member of the first binding pair is a species-specific antibody.
[58]. The kit according to any of [52] to [57], wherein the lateral flow device is a test strip.
[59]. The kit according to [58], wherein the test strip is a nitrocellulose membrane.
[60]. The kit according to any of [38] to [59], wherein the target DNA sequence is from a pathogen.
[61]. The kit according to [60], wherein the pathogen is an organism from the *Kinetoplastea* class.
[62]. The kit according to [61], wherein the organism from the *Kinetoplastea* class is *Leishmania.*
[63]. The kit according to [62], wherein the *Leishmania* is *Leishmania infantum.*
[64]. The kit according to any of [61] to [63], wherein the target DNA sequence is a sequence of the kinetoplast.
[65]. The kit according to [64], wherein the pair of primers capable of specifically amplifying the target DNA sequence are primers of sequence SEQ ID NO: 1 and SEQ ID NO: 2.
[66]. The kit according to any of [44] to [65], wherein the endogenous amplification control DNA sequence is a sequence of the 18S rRNA gene.
[67]. The kit according to [66], wherein the 18S rRNA gene is from dog.
[68]. The kit according to [67], wherein the pair of primers capable of amplifying the endogenous amplification control DNA sequence are primers of sequence SEQ ID NO: 3 and SEQ ID NO: 4.
[69]. An *in vitro* method for diagnosing an infection by a pathogen in a subject comprising detecting the presence of a target DNA sequence from said pathogen in a biological sample of said subject by a method according to any of [1] to [37].
[70]. An oligonucleotide whose sequence is selected from the group consisting of SEQ ID NO: 1, SEQ ID NO: 2, SEQ ID NO: 3 and SEQ ID NO: 4.
[71]. The oligonucleotide according to [70], wherein said oligonucleotide is labelled at the 5' end with a gold nanoparticle or with a first member of a binding pair.

The following examples are provided as merely illustratives and are not to be construed as limiting the scope of the invention.

### EXAMPLES

### MATERIALS AND METHODS

### 1. Leishmania/18S isothermal amplification

### 1.1. Design and screening of primer sets for Leishmania assay

Initially, 3 candidate primers for kinetoplast and 8 candidate primers for Intergenic Spacer region (ITS1) of *Leishmania* were designed following recommended conditions [optimum length between 30-35nt; without long tracks of guanines at the 5' end (first 3-5 nucleotides) and 'unusual' sequence elements such as homopolymers; with a GC content between 30% and 70% and preferably with cytidines at the 5' end and guanines and cytidines at the 3' end of the primer (last 3 nucleotides) to improve performance]. Amplicon length must not exceed about 500bp, and ideally must be between 100-200bp.

All possible combinations of primers were tested using standard conditions of TwistAmp® kits (TwistDX) (37°C of reaction temperature; magnesium-acetate concentrations of 14mM; shaking 4' after initiation of the reaction; 480nM of each primer).

Parameters that can be modified by the user [reaction temperature (37°-42°C), magnesium concentration (12mM and 20mM), agitation regime (3'- 6' after initiation of the reaction) and primer concentrations (150nM-600nM)] were adjusted to obtain the best performance.

This first step generated 4 sets of primers used in simplified standard conditions (37°C of reaction temperature, 14mM of magnesium concentration, 480nM of primer concentration, without agitation 4' after starting the reaction and with a reaction time of 20').

The kinetoplast region was chosen due to the higher intensity obtained in an agarose gel after selected target amplification.

### 1.2. Secondary primer screen for Leishmania assay

To improve the performance of the *Leishmania* kinetoplast assay a 'second generation' of primers was designed by creating variants of the best primer set identified in the first step (moving 1 base pair around the initial primer) and re-screened the new candidates to improve amplification performance.

Primers set selected at last were:
K6F CTTTTCTGGTCCTCCGGGTAGGGGCGTTCTG (31 bp) (SEQ ID NO: 1)
K3R CCACCCGGCCCTATTTTACACCAACCCCCAGTTTCCC (37bp) (SEQ ID NO: 2)
that amplified a fragment of approximately 140 base pair length.

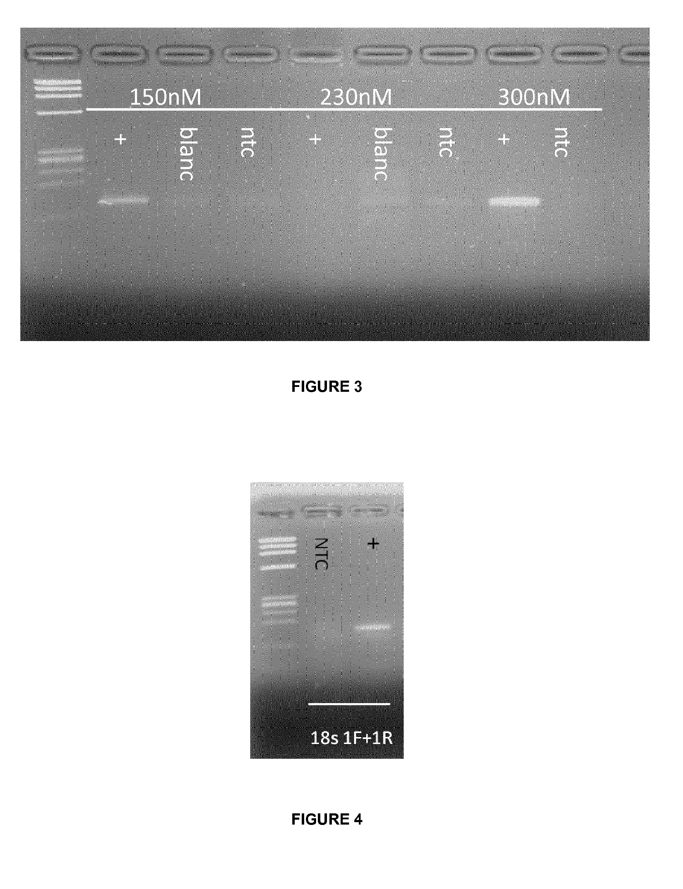

Primer concentration was adjusted to 300nM and the reaction time was decreased up to 10'. Under these conditions the limit of detection was <1 parasite in the reaction (analyzed in agarose gel electrophoresis) (Figure 3).

### 1.3. Design and optimization of an endogenous control

6 candidate primers for 18S ribosomal RNA gene were designed. This gene was chosen because it has multiple copies in the genome as it occurs with kinetoplast of Leishmania.

All possible combinations of primers were tested using standard conditions of TwistAmp® kits (300nM of primer concentration and 10' of amplification time) and the primer set definitively chosen was:
Primer 18s 1F CTGCGAATGGCTCATTAAATCAGTTATGGTTCC (SEQ ID NO: 3)
Primer 18s 1R CTGACCGGGTTGGTTTTGATCTGATAAATGCACGC (SEQ ID NO: 4)
that amplified a fragment of 168 base pair length (Figure 4).

### 1.4. Isothermal amplification assay conditions

Finally, a multiplex isothermal was optimized in which primer concentration of the endogenous control assay was limited to 150nM to avoid a loss of sensitivity when low targets of *Leishmania* were present.

To obtain double labelled amplicons [FITC-BIOTIN (for *Leishmania* assay) and FITC-DIGOXIGENIN (for 18S assay)] Twist proposes a complicate protocol (nfo protocol) that adds an additional internal probe to the assay. In this case it was impossible to design an internal probe for *Leishmania* assay due to the high polymorphism of the region.

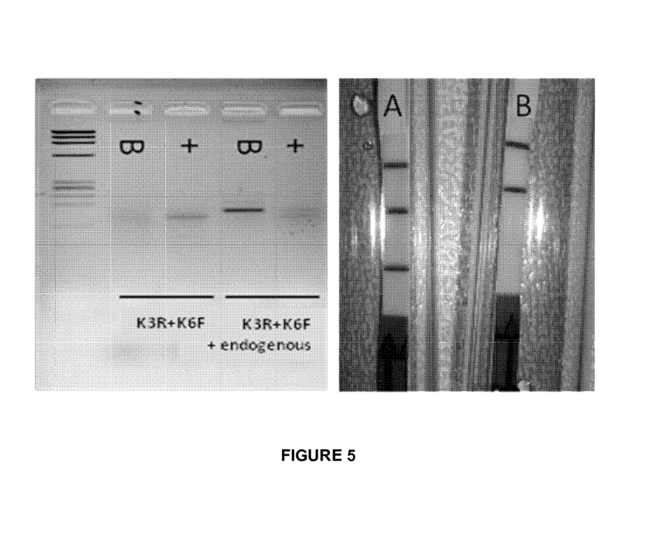

Multiplex amplification with labelled primers using K6F-FITC and K3R-Biotin for *Leishmania* and 18s-1 F-FITC and 18s-1 R-Digoxigenin for endogenous control was performed using 14mM of magnesium concentration, 300 nM of *Leishmania* primers, 150nM of 18s primers and 37°C of reaction temperature during 10 minutes without agitation (Figure 5).

### 2. Lateral flow detection

### 2.1. Commercial LF Milenia® HybriDetec 2T

Lateral flow detection was performed using the Milenia® HybriDetec 2T, a universal lateral flow dipstick for simultaneous detection of two different analytes labelled with FITC, biotin and digoxigenin.

Detection was performed using 5µl of 1/20 dilution in milli-Q™ water of the amplification product in 100µl of the running buffer, as manufacturer's instructions.

### 2.2. Enhanced lateral flow assay using secondary antibodies labelled with gold nanoparticles

### 2.2.1. Preparation of gold nanoparticles

Gold nanoparticles (AuNP) 20 nm sized and stabilized by citrate were prepared using the Turkevich's method (Turkevich et al. Discuss. Faraday Soc. 1951, 11:55-75). Briefly, 50 mL aqueous solution of 0.1% HAuCl₄ were heated to boiling and vigorously stirred in a 250 mL round-bottom flask; 1.25 mL of sodium citrate 1% were added quickly to this solution. Boiling was continued for additional 10 min. The solution was cooled to room temperature with a continuous stirring. The colloids were stored in dark bottles at 4° C. All glassware used in this preparation was previously cleaned in aqua regia overnight and rinsed with double distilled H₂O. Reflux was used for all the procedure.

### 2.2.2. Gold nanoparticles modification with antibodies: preparation of the double antibody solution

First, the pH of the AuNPs suspension was corrected to pH 9 with 0.1 M borate buffer. Then, 100 µL of a 100 µg/mL anti-goat IgG aqueous solution were added to 1.5 mL of the AuNPs suspension. The resulting solution was incubated for 20min at 650rpm. Then, 100µL of 1mg/mL BSA aqueous solution were added and the stirring was continued for other 20min at 650rpm. Finally, the solution was centrifuged at 14000 rpm and 4°C.

The supernatant was removed and the pellet of AuNP/anti-goat IgG was resuspended in 300 µL of 3 µg/mL anti-FITC IgG solution in BB 2mM pH 7.4, 10% sucrose. In this way, a suspension containing both anti-FITC IgG and AuNPs/anti-goat IgG was obtained and was immediately used for conjugate pad preparation.

### 2.2.3. Preparation of the strips

1 mg/mL solution of anti-goat IgG and streptavidin in PB 10 mM, pH 7.4 were spotted onto the detection pad at a dispensing rate of 0.05 µL/mm using an IsoFlow reagent dispensing system to form the control and test line, respectively. For the detection of the endogenous control, an additional line with 1 mg/mL of anti-digoxigenin was also spotted. Then, the detection pad was dried at 37°C for 1 h. After that, the membrane was blocked using a 2% BSA aqueous solution for 5 minutes. Finally, the membrane was washed for 15 minutes using PB (5mM pH 7.4), 0.05% SDS and dried at 37°C for 2h.

The sample pad was prepared by dipping the strip into 10mM PBS, 5% BSA and 0.05% Tween®-20 and drying it at 60°C for 2h. The conjugate pad was prepared dipping it into the previously prepared double antibody solution (anti-FITC IgG and AuNP/anti-goat IgG) and drying it under vacuum for 1 h.

The different pads were sequentially laminated 2 mm with each other and pasted onto the adhesive backing card in the following order: detection, conjugation, sample and absorbent pads. Finally, the strips were cut 7 mm wide and stored in dry conditions at 4°C until their use up to a week.

### 2.2.4. Lateral-flow assay procedure

Isothermally amplified DNA sample solutions labelled with FITC/biotin and FITC/digoxigenin (provided by Vetgenomics) were first diluted in tris buffer saline 10mM Trizma®-HCl pH 7.6 and 0.05% Tween®-20 (TBST) at different dilution factors. TBST without analyte was considered as blank. Typical assay consisted in mixing 10 µL of the diluted sample (different dilution factors in tris buffer saline 10mM Tris-HCl pH 7.6 and 0.05% Tween-20 (TBST) were assayed: 1:100, 1:250: 1:500, 1:1250, 1:2500, 1:5000) with 200 µL of TBST, immersing the strip into this solution and keeping it for 10 min until the flow is stopped. Then 200 µl of TBST were dispensed in order to wash away the excess of the double antibody solution. Three replicates of each sample were done and intensities of lines were read with the strip reader so as to obtain the corresponding calibration curve.

### 3. Electrochemical detection

### 3.1. Screen-printed carbon electrodes (SPCE) fabrication

The electrochemical transducers used for the nanoparticle label detection were homemade screen-printed carbon electrodes (SPCEs), consisting of three electrodes: working electrode WE, reference electrode RE and counter electrode CE in a single strip. The full size of the sensor strip was 29mm x 6.7mm, and the WE diameter was 3mm. The fabrication of the SPCEs was carried out in three steps. First, a graphite layer was printed onto the polyester sheet, using the screen-printing machine with the stencil (where it is the electron pattern). After curing for 15 minutes at 95°C, an Ag/AgCl layer was printed and cured for 15 minutes at 95°C. Finally, the insulating ink was printed and cured at 95°C for 20 minutes. Figure 6 shows details of this procedure as well as of the obtained sensors.

### 3.2. Gold nanoparticles synthesis

Gold nanoparticles were synthesized as described in section 2.2.1.A scheme of the AuNPs synthesis procedure as well as a TEM image is shown in Figure 7.

### 3.3. Gold nanoparticles conjugation with thiol-modified primers

The conjugation of AuNPs to primers already modified with thiol groups was performed adapting the procedure disclosed in Storhoff et al. [Storhoff, JJ., Elghanian, R., Mucic, RC., Mirkin, CA., Letsinger, RL. One-pot colorimetric differentiation of polynucleotides with single base imperfections using gold nanoparticle probes. J. Am. Chem. Soc. 120 (1998): 1959-1964]. 190 µL of AuNPs suspension were mixed with 10 µL of 1500 µg/mL thiolated primer solution and incubated for 20h at 25 °C with agitation (250 rpm) (final concentration of primer: 75 µg/mL). After that, this solution was added to 50 µL of 10 mM phosphate buffer (pH 7) / 0.1 M NaCl and allowed to stand for 44h. Finally, a centrifugation at 14000 rpm for 20 min at 4°C was carried out, and AuNPs/primer conjugates were reconstituted in 200 µL of milli-Q™ water (Figure 7b).

### 3.4. Gold nanoparticles conjugation with anti-FITC antibodies

The conjugation of AuNPs to anti-FITC antibodies, was performed according to the following procedure [Ambrosi, A., Castañeda, MT., Killard, A.J., Smyth, MR., Alegret, S., Merkoçi, A. Double-codified gold nanolabels for enhanced immunoanalysis. Analytical Chemistry, 79 (2007) 5232-5240]: 2 mL of AuNPs suspension were mixed with 100 µL of 100 µg/mL of antibody solution and incubated for 20 min at 25 °C with agitation (650 rpm). Subsequently, a blocking step with 150 µL of 1 mg/ml BSA, incubating for 20 min at 25 °C with agitation (650 rpm) was undertaken. Finally, a centrifugation at 14000 rpm for 20 min at 4 °C was carried out, and AuNPs/anti-FITC conjugates were reconstituted in milli-Q™ water.

### 3.5. Zeta potential measurements

A 1 µL suspension of AuNPs, AuNPs/primer and of AuNPs/amplified DNA was diluted in 1 mL of PBS buffer, vortexed, and transferred into a 4 mL polystyrene cuvette (FB55143, Fisher Scientific). The data were collected and analyzed with the Dispersion Technology software 4.20 (Malvern) producing diagrams for the zeta potential as a distribution versus total counts.

### 3.6. Magnetic capturing of the isothermally amplified product already labelled with gold nanoparticles

The isothermally amplified product was already labelled with gold nanoparticles, since one of the primers was connected with AuNPs as detailed in section 3.3. 10 µL of the streptavidin-modified magnetic beads (MBs) suspension (100 µg) were mixed with 90 µL of B&W buffer. After washing 2 times with B&W, the suspension was re-suspended in 50 µL of B&W and 50 µL of the isothermally amplified product (labelled with thiol and AuNPs) were added and then incubated for 30 min at 25° C with agitation (650 rpm). During this incubation, the isothermally amplified product was captured by the MBs through the streptavidin-biotin interaction. "Positive" (amplified DNA from dogs with Leishmania) and "blank" samples (amplified DNA from healthy dogs) were assayed.

The rest of reagents in solution (excess of primers, etc.) were removed by washing with B&W (2 times), PBS buffer (2 times) and milli-Q™ water in order to remove the excess of AuNPs-FITC. The final complex was re-suspended in milli-Q™ water.

A scheme of this procedure together with a picture of the magnetic separation platform and a SEM image of the MBs is shown in Figure 8 (steps 2-3).

### 3.7. Integration of magnetic beads before amplification

MBs were connected with one of the primers through the streptavidin-biotin interaction, following this experimental procedure: (i) incubation of the MBs (50 µL; 1 mg/mL) with biotinylated primer (15 µL; 100 µM) (30 min; 25°C); (ii) washing and re-suspending in 15 µL of water; (iii) addition of 2.4 µL of the complex MBs/primer to the isothermal amplification reagents mixture. A scheme of the experimental procedure for the detection of isothermally amplified DNA using primers labelled with gold nanoparticles and MBs is shown in Figure 9.

### 3.8. Electrochemical detection

25 µL of the MBs complex suspension were placed on the working area of the SPCE (connected to the potentiostat and where it was previously attached a hard plastic base with a magnet on the reverse side of the working area). After 30 seconds, 25 µL of 2M HCl solution were added and a potential of +1.35 V was applied during 1 min (electrochemical pre-treatment). After that, a potential of -1.00 V was applied during 100 seconds in chronoamperometric mode. Under these conditions, the H⁺ ions were reduced to H₂ thanks to the catalytic effect of the AuNPs labels. The absolute value of the current registered at 100 seconds was considered as the analytical signal, being this value proportional to the quantity of AuNPs and, consequently, to the concentration of isothermally amplified product. A scheme of the electrocatalytic reduction of H⁺ ions by the AuNPs is shown in the last step of Figure 8.

### Results

### Specificity of the assay with commercial lateral flow detection

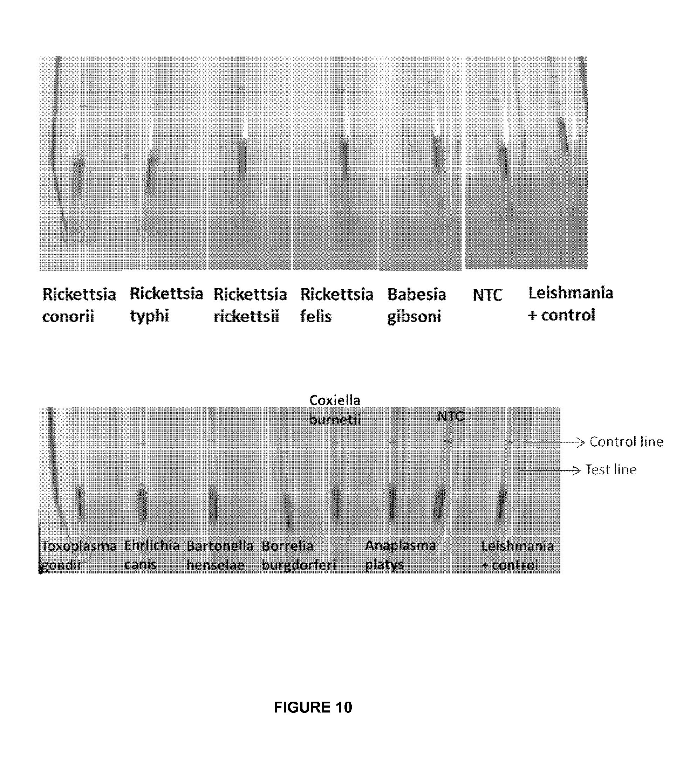

Specificity was tested (i) *in silico,* (ii) sequencing the positive control and (iii) performing a cross-amplification study in which amplification with specific primers was performed over all the positives of other pathogens that could be in co-infection, mostly CVBD (Canine Vector Borne Diseases) (Figure 10). Expected results are amplification of the specific positive sample and no amplification of any of the other pathogens tested. DNA of positive control of CVBD was obtained from commercial slides from Megacor coated with cells infected with the pathogens (*Ehrlichia canis, Babesia gibsoni, Rickettsia rickeftsii, Rickettsia conorii, Rickettsia felis, Rickettsia typhi, Bartonella henselae* and *Anaplasma platys)* or commercial DNA from Genekam Biotechnology AG *(Borrelia burgdorferi, Coxiella burnetii* and *Toxoplasma gondii).*

The assay of the inventors has 100% specificity.

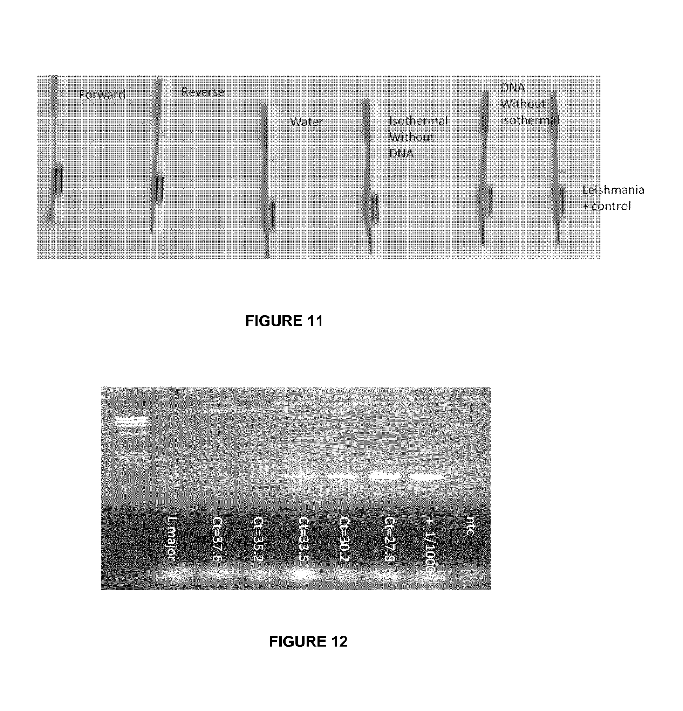

It was also demonstrated lateral flow specificity analyzing only one of the labelled primers with water (forward or reverse), water, isothermal amplification without DNA and DNA alone without isothermal amplification (Figure 11).

### Quantitative real time Leishmania PCR comparison

Results were compared with those obtained by quantitative real time amplification using the PCR described by Francino et al (Francino et al. 2006. Veterinary Parasitology 137(3-4):214-221) (Table I).

**Table I. Results of parasitemia of different samples obtained by quantitative real time PCR (qPCR).**

| sample | qPCR Ct | Parasites/µl DNA | Parasites/ml blood |
|---|---|---|---|
| 1 | 37.6 | 0.0018 | 0.5 |
| 2 | 35.2 | 0.0074 | 1.88 |
| 3 | 33.5 | 0.0206 | 5.15 |
| 4 | 30.2 | 0.146 | 36.5 |
| 5 | 27.8 | 0.606 | 151.68 |

An analysis by isothermal amplification of the same DNAs analysed by qPCR with different parasitemia levels was performed. Isothermal amplification amplified without problems DNAs with 0.02 parasites/µl detected by agarose gel electrophoresis (Figure 12).

### Quantitative analysis and estimation of the limit of detection

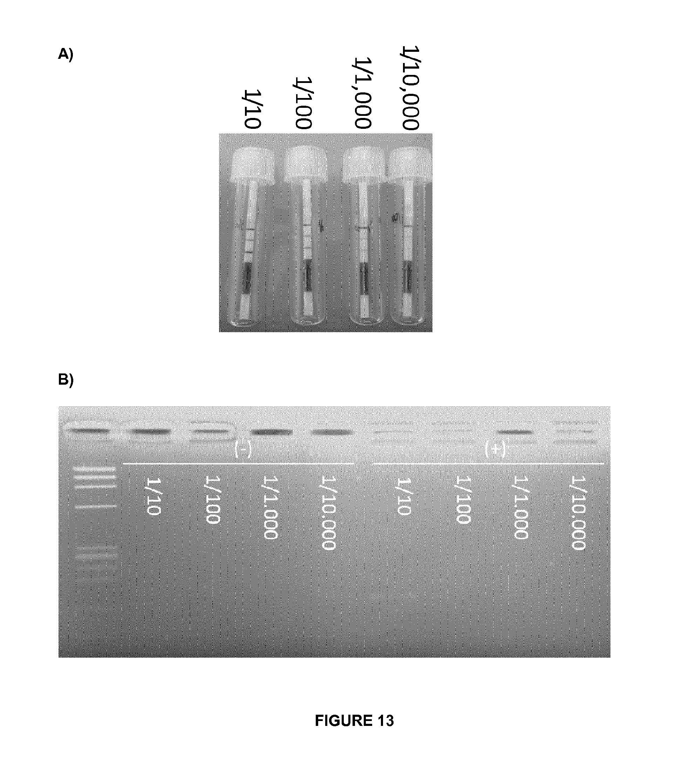

Serial dilutions of an isothermal amplified product starting with 1.5 parasites in the isothermal reaction were performed. Results showed that lateral flow (Figure 13A) is more sensitive than agarose gel electrophoresis (Figure 13B) for detection. Three fold of sensitivity (1/10.000 versus 1/10) was gained (Figure 13).

The limit of detection was estimated by evaluating amplified products prepared from samples containing different concentrations of spiked parasites. 20ng of dog DNA with different amounts of parasite were spiked to finally perform the isothermal amplification starting with 20 parasites in the assay, followed by 4 parasites, 2 parasites, 0.4 parasites, 0.2 parasites, 0.04 parasites and 0.02 parasites. The limit of detection with the commercial lateral flow assay was of 0.2 parasites in the isothermal amplification.

### Enhanced lateral flow with secondary antibody improves the limit of detection

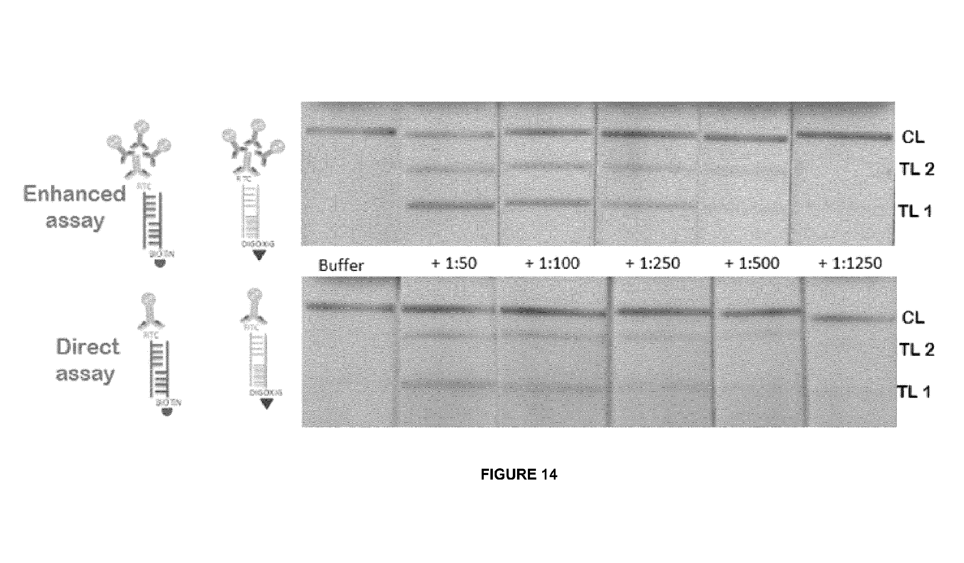

A novel design of Lateral Flow Assay (LFA) based on the use of secondary antibodies in the conjugate pad was successfully accomplished, enhancing the intensity of the signal in the test line and consequently the sensitivity of the assay, allowing detecting products diluted up to 1:1250. As it is shown in Figure 14 (upper part), the additional test line was clearly visualized. Furthermore, the intensity of color in the control line remained constant while the corresponding to the two test lines decreased when the dilution factor was increased, demonstrating that the amplification procedure worked properly.

The results were also compared with those obtained using the direct assay (without secondary antibodies; Figure 14-lower part) observing again the advantageous effect of the enhanced strategy, ensuring now that false negatives are avoided.

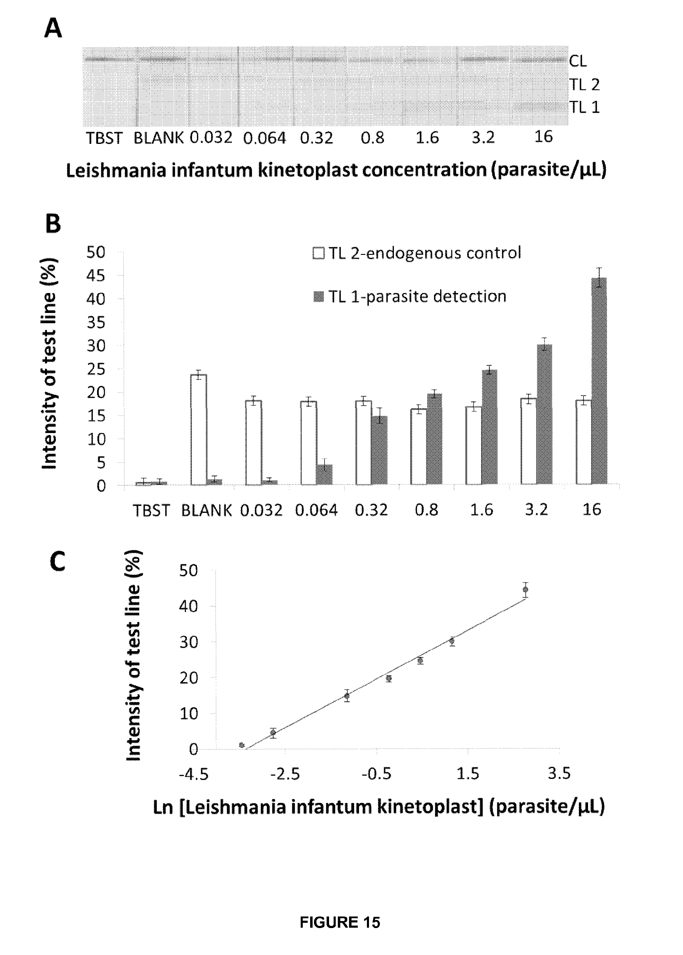

The sensitivity of the enhanced assay was also evaluated in terms of parasite concentration. Dog DNA samples with different quantities of spiked *Leishmania* parasites in the range 0.032-16 parasites/µL were assayed, containing the endogenous control (figure 15A and 15B). A good correlation between the parasite concentration and the analytical signal, adjusted to a logarithmic curve was observed, as shown in figure 15C. A detection limit of 300 parasites/mL of sample was estimated, with also a very good reproducibility (RSD: 4.5%).

### Zeta potential characterization of AuNP-labelled primers and amplified DNA

Zeta potential is an easily measurable technique, recently reported as an efficient tool for the monitoring and analysis of modifications on the surface of NPs, with minimal sample preparation [F. Thielbeer, K. Donaldson, M. Bradley, Bioconj. Chem. 2011, 22, 144-150]. It has been used to obtain information concerning the particle surface charge, chemical modifications and also stability of colloid suspensions. A high zeta potential (positive or negative; typically higher than 10 mV) confers stability since the dispersion resists aggregation.

Since ssDNA is negatively charged, the conjugation of AuNPs with ssDNA should give rise to negative charged conjugates which would shift the Z potential to more negative values.

All these characteristics make zeta potential an ideal technique for the characterization of both the primers and the final amplified DNA labelled with AuNPs.

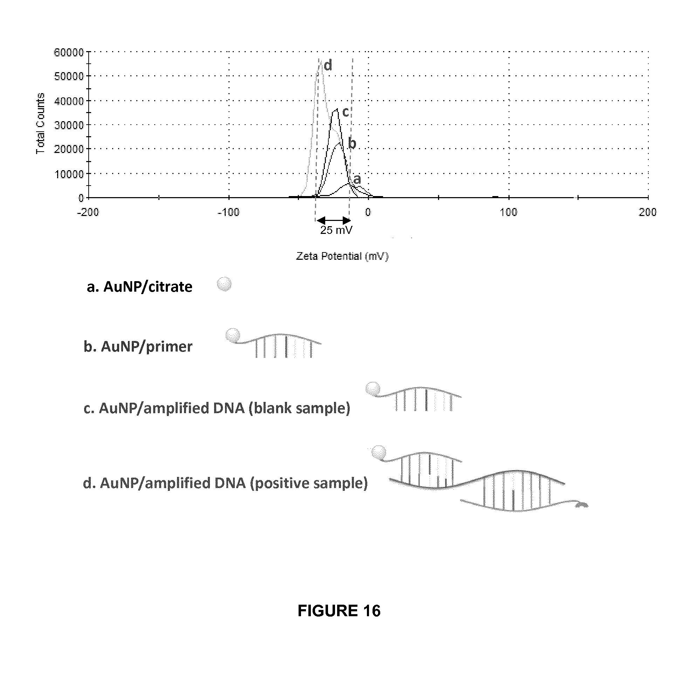

As can be observed in Figure 16, a gradual shift to more negative values was observed in the Zeta potential of AuNPs when higher was the coverage degree by ssDNA. This suggests that AuNPs were being loaded with negative charged molecules that is the case of ssDNA. First, a shift from the value of the non-modified AuNPs (a) was observed for the AuNP/primer (b) indicating that the primer was correctly connected with the AuNP. After the amplification, this shift was up to 25 mV (d), suggesting the covering of the AuNPs with the amplified DNA that has more negative charges. No significant shift was observed for a control amplification assay performed with a blank sample (c), also corroborating the specificity of the system.

### Electrochemical detection of DNA amplified using AuNP-labelled primers.

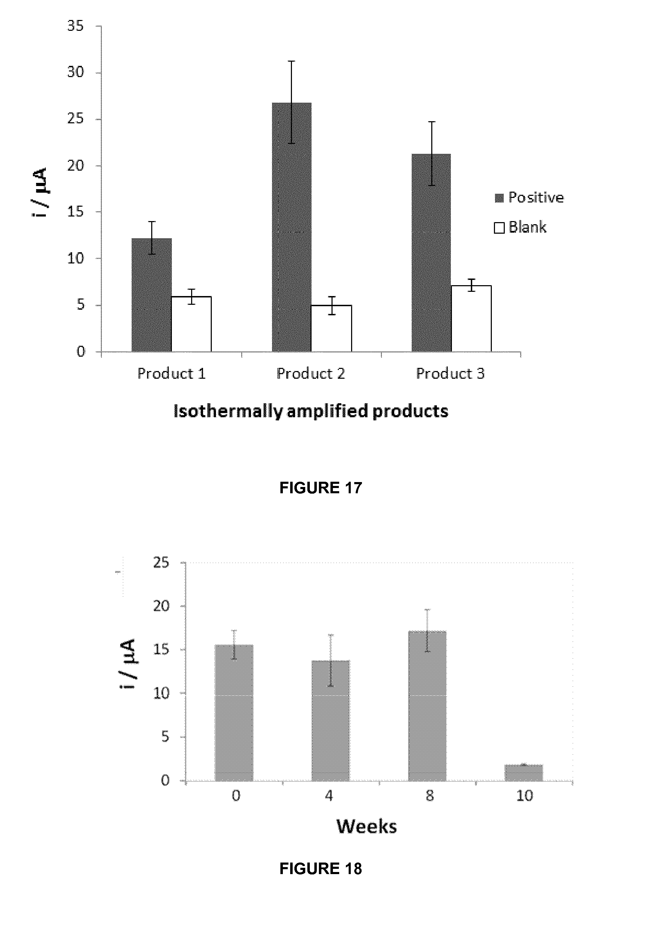

The AuNP-labelled amplified products were electrochemically detected following the above detailed experimental procedure. The electrochemical method exhibited a good reproducibility and sensitivity, allowing to perfectly discriminate amplified DNA from dogs without *Leishmania,* demonstrating the specificity of both the amplification procedure and the electrochemical detection, as shown in Figure 17.

The stability of the primer labelled with AuNPs is a very important parameter that has been evaluated. A robust method needs to have disposable primers ready to be used in a similar way than the commercially available primers labelled i.e. with biotin or FITC.

In order to study such stability, a conjugate of AuNP/primer was prepared, stored at 4°C (protected from light) and used for different isothermal amplifications performed after several weeks. As shown in Figure 18, the amplification worked well for conjugates prepared up to 8 weeks before. However, any signal was obtained after 10 weeks), indicating that probably the connection between the primer and the AuNPs was broken.

### Electrochemical detection of DNA amplified using both AuNP and MB-labelled primers

The integration of the MBs also in the primers used for the isothermal amplification was also evaluated, so as to highly simplify this experimental procedure, as detailed in section 3.7.

MBs of two different sizes (the standard ones of 2.8 µm and also smaller commercially available ones of 1 µm) and two different kind of primers were evaluated: the same than used before and also a longer one, containing the same amplification region but including also an spacer, that is a tail of oligonucleotides that helps to keep the magnetic bead far from the amplification zone.

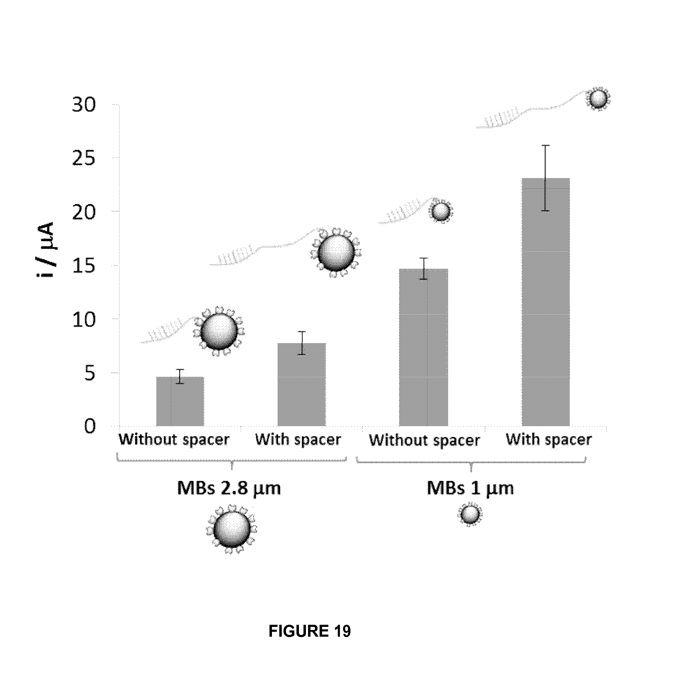

The isothermal amplification was performed using one primer labelled with AuNPs and the other one labelled with the magnetic bead following one of these strategies (different sized MBs and primers) (Figure 19). In this case, the experimental procedure for the measurement after the amplification is enormously simplified since only a washing step of few seconds is necessary immediately before the electrochemical measurement.

Results seem to indicate that the amplification doesn't work using the bigger MBs in combination with the short primer since the currents recorded are in the levels of the background. The results improved a little bit when the use of longer primers, with the spacer, was introduced. When the smaller MBs and the short primer were tried, the amplification worked in a higher extent, and this was highly improved when it was used in combination with the primers with spacer. So this suggests that the bigger MBs are hindering the amplification, probably due to the beads deposition during the amplification procedure. Furthermore, it is better to have the amplification sequence of the primer not in direct contact with the bead so as to facilitate the amplification.

## Claims

1. An *in vitro* method for detecting a target DNA sequence in a sample comprising the following steps:
a) submitting the sample DNA to a recombinase-polymerase isothermal nucleic acid amplification in the presence of a pair of primers capable of specifically amplifying the target DNA sequence wherein the first primer is labelled at the 5' end with a gold nanoparticle and the second primer is labelled at the 5' end with a first member of a binding pair, and
b) measuring the electrochemical signal produced by the gold nanoparticles forming part of the amplification products obtained in step a) using a screenprinted carbon electrode after capture of the amplification products on the electrode using magnetic beads conjugated to a second member of a binding pair,
wherein the detection of an electrochemical signal superior to the background signal indicates the presence of the target DNA sequence in the sample.

2. An *in vitro* method for detecting a target DNA sequence in a sample comprising the following steps:
a) submitting the sample DNA to a recombinase-polymerase isothermal nucleic acid amplification in the presence of a pair of primers capable of specifically amplifying the target DNA sequence wherein the first primer is labelled at the 5' end with a first member of a first binding pair and the second primer is labelled at the 5' end with a first member of a second binding pair, and
wherein said first members of the first and second binding pairs do not substantially cross-react with any second member of any different binding pair, and
b) measuring the electrochemical signal produced by the gold nanoparticles forming part of the amplification products obtained in step a) using a screenprinted carbon electrode after the first member of a first binding pair is bound to a second member of a first binding pair conjugated to a gold nanoparticle and after capture of the amplification products on the electrode using magnetic beads conjugated to a second member of a second binding pair
wherein the detection of an electrochemical signal superior to the background signal indicates the presence of the target DNA sequence in the sample.

3. The method according to any of claims 1 or 2, wherein:
(i) if the detection is carried out by a method according to claim 1, the second primer labelled at the 5' end with a first member of a binding pair is bound to the second member of the binding pair conjugated to magnetic beads before step a) takes place, and wherein said magnetic beads have a size lower than 2.8 µm and are separated from the region of the primer that recognizes the target DNA sequence by a polynucleotide spacer, and
(ii) if the detection is carried out by a method according to claim 2, the second primer labelled at the 5' end with a first member of a second binding pair is bound to the second member of the second binding pair conjugated to magnetic beads before step a) takes place, and wherein said magnetic beads have a size lower than 2.8 µm and are separated from the region of the primer that recognizes the target DNA sequence by a polynucleotide spacer.

4. The method according to any of claims 1 to 3, wherein:
(i) if the detection is carried out by a method according to claim 1, the second member of the binding pair conjugated to magnetic beads is added after step a), and
(ii) if the detection is carried out by a method according to claim 2, the second member of the second binding pair conjugated to magnetic beads is added after step a).

5. An *in vitro* method for detecting a target DNA sequence in a sample comprising the following steps:
a) submitting the sample DNA to a recombinase-polymerase isothermal nucleic acid amplification in the presence of
(i) a first pair of primers capable of specifically amplifying the target DNA sequence wherein the first primer of the first pair of primers is labelled at the 5' end with a first member of a first binding pair and the second primer of the first pair or primers is labelled at the 5' end with a first member of a second binding pair and
(ii) a second pair of primers capable of amplifying an endogenous amplification control DNA sequence present in the sample wherein the first primer of the second pair of primers is labelled at the 5' end with said first member of said first binding pair and the second primer of the second pair of primers is labelled at the 5' end with a first member of a third binding pair
wherein said first members of the first, second and third binding pairs do not substantially cross-react with any second member of any different binding pair and
b) performing a lateral flow assay for the detection of the amplified DNA obtained in step a), wherein
the amplified DNA obtained in step a) is added to the sample pad of a lateral flow device and
a second member of the first binding pair associated to a marker capable of generating a colorimetric signal is immobilized on the conjugate pad of a lateral flow device
and wherein
the lateral flow device comprises a first area wherein a second member of the second binding pair has been immobilized, a second area wherein a second member of the third binding pair has been immobilized and a third area wherein a reagent capable of specifically binding to the second member of the first binding pair has been immobilized
wherein the order of the first area and the second area in the lateral flow device is interchangeable
wherein the detection of a colorimetric signal in the first, second and third area of the lateral flow device indicates the presence of the target DNA sequence in the sample, or wherein the absence of a colorimetric signal in the first area and the detection of a colorimetric signal in the second and third area of the lateral flow device indicates the absence of the target DNA sequence in the sample.

6. The method according to any of claims 1 to 5, wherein:
(i) if the detection is carried out by a method according to claim 1, the first member of a binding pair is biotin and the second member of a binding pair is streptavidin, and
(ii) if the detection is carried out by a method according to claim 2 or by a method according to claim 5, the first member of the first binding pair is fluorescein isothiocyanate (FITC) and the second member of the first binding pair is an antibody specific for said antigen and, wherein the first member of the second binding pair is biotin and the second member of the second binding pair is streptavidin.

7. The method according to any of claims 5 or 6, wherein the marker capable of generating a colorimetric signal is a gold nanoparticle.

8. The method according to any of claims 5 to 7, wherein the marker capable of generating a colorimetric signal is conjugated to the second member of the first binding pair.

9. The method according to any of claims 5 to 7, wherein the marker capable of generating a colorimetric signal is conjugated to an antibody which binds specifically to the second member of the first binding pair.

10. The method according to any of claims 1 to 9, wherein the target DNA sequence is from *Leishmania.*

11. The method according to any of claims 1 to 10, wherein the target DNA sequence is a sequence of the kinetoplast.

12. The method according to any of claims 5 to 11, wherein the endogenous amplification control DNA sequence is a sequence of the 18S rRNA gene.

13. A kit selected from the group consisting of:
a) a kit comprising a pair of primers capable of specifically amplifying a target DNA sequence wherein the first primer is labelled at the 5' end with a gold nanoparticle and the second primer is labelled at the 5' end with a first member of a binding pair, and the first member of a binding pair is bound to a second member of a binding pair conjugated to magnetic beads, and wherein said magnetic beads have a size lower than 2.8 µm and are separated from the region of the primer that recognizes the target DNA sequence by a polynucleotide spacer;
b) a kit comprising:
(i) a pair of primers capable of specifically amplifying a target DNA sequence wherein the first primer is labelled at the 5' end with a gold nanoparticle and the second primer is labelled at the 5' end with a first member of a binding pair, and
(ii) a second member of a binding pair conjugated to magnetic beads, and
c) a kit comprising:
(i) a first pair of primers capable of specifically amplifying a target DNA sequence wherein the first primer of the first pair of primers is labelled at the 5' end with a first member of a first binding pair and the second primer of the first pair of primers is labelled at the 5' end with a first member of a second biding pair,
(ii) a second pair of primers capable of amplifying an endogenous amplification control DNA sequence wherein the first primer of the second pair of primers is labelled at the 5' end with said first member of said first binding pair and the second primer of the second pair of primers is labelled at the 5' end with a first member of a third binding pair,
wherein said first members of the first, second and third binding pairs do not substantially cross-react with any second member of any different binding pair,
(iii) a recombinase,
(iv) a single-stranded DNA binding protein and
(v) a strand-displacing DNA polymerase.

14. An *in vitro* method for diagnosing an infection by a pathogen in a subject comprising detecting the presence of a target DNA sequence from said pathogen in a biological sample of said subject by a method according to any of claims 1 to 12.

15. An oligonucleotide whose sequence is selected from the group consisting of SEQ ID NO: 1, SEQ ID NO: 2, SEQ ID NO: 3 and SEQ ID NO: 4.


SEQUENCE LISTING

<110>  VETGENOMICS, S.L.

<120>  METHODS FOR DETECTING TARGET DNA SEQUENCES

<130>  P10398EP00

<160>  5

<170>  PatentIn version 3.5

<210>  1
<211>  31
<212>  DNA
<213>  Artificial Sequence

<220>
<223>  K6F primer

<400>  1
cttttctggt cctccgggta ggggcgttct g                                    31


<210>  2
<211>  37
<212>  DNA
<213>  Artificial Sequence

<220>
<223>  K3R primer

<400>  2
ccacccggcc ctattttaca ccaaccccca gtttccc                              37


<210>  3
<211>  33
<212>  DNA
<213>  Artificial Sequence

<220>
<223>  18s 1F primer

<400>  3
ctgcgaatgg ctcattaaat cagttatggt tcc                                  33


<210>  4
<211>  35
<212>  DNA
<213>  Artificial Sequence

<220>
<223>  18s 1R primer

<400>  4
ctgaccgggt tggttttgat ctgataaatg cacgc                                35


<210>  5
<211>  15
<212>  DNA

<213>  Artificial Sequence

<220>
<223>  polynucleotide spacer

<400>  5
atatatatat atata                                                        15